# EUROPEAN PATENT APPLICATION

(11) **EP 4 678 086 A1**
(43) Date of publication of application: **14.01.2026**
(21) Application number: 23949943.7
(22) Date of filing: 31.08.2023
(51) Int. Cl.: A61B 1/00

(54) **ENDOSCOPE AND ENDOSCOPE OBJECTIVE LENS**

(71) Applicant: Macrolux Medical Technology Co., Ltd., Shenzhen, Guangdong 518132 (CN)
(72) Inventor: WANG, Lei, Shenzhen, Guangdong 518132 (CN)
(74) Representative: Manitz Finsterwald Patent- und Rechtsanwaltspartnerschaft mbB
(86) International application number: PCT/CN2023/116246
(87) International publication number: WO 2025/043632

(57) **Abstract**

The present disclosure relates to the field of endoscopy, specifically to an endoscope objective lens. In the optical system of the objective lens, a first lens, a second lens, an aperture stop, a third lens and a fourth lens are sequentially arranged from the image side to the object side along the optical axis. The first and fourth lenses have negative optical power, while the second and third lens have positive optical power. The chief ray height h11 on the object side surface of the first lens and the chief ray height h42 on the image side surface of the fourth lens satisfy: 0.65 ≤ h11/h42 ≤ 1.35; and/or the effective optical diameter D11 of the object side surface of the first lens and the effective optical diameter D42 of the image side surface of the fourth lens satisfy: 0.75 ≤ D11/D42 ≤ 1.25. This configuration enables approximate symmetry between the first and fourth lens, resulting in approximately symmetric light paths through the two lenses. Consequently, partial mutual cancellation of aberrations occurs, thereby reducing aberration correction difficulty and tolerance sensitivities of optical surfaces.

## Description

### TECHNICAL FIELD

The present disclosure relates to endoscopes, and more particularly to the objective lens of an endoscope.

### BACKGROUND OF THE DISCLOSURE

Endoscopic devices features compact dimensions and direct imaging, and are now widely applied in diverse fields. Endoscopes can access body cavities with no or minimal invasion, thus enabling accurate diagnosis or treatment through observation of the endoscopic images.

Given the restricted dimensions of natural orifices in human anatomy, even surgical incisions necessitate minimization of invasion extent. Consequently, endoscopes are subject to rigorous dimensional constraints, particularly those employed for observation of: the gastrointestinal tract (including hepatobiliary system), bronchi, otorhinolaryngological cavities, genitourinary system, and uterus.

The diameter of an endoscope must be maintained within compact dimensions, and correspondingly, the objective optical system at its distal end is likewise constrained. During examination of narrow tortuous lumens such as the bladder and ureter (components of the lower urinary tract), clinical protocols typically require deflection exceeding 90° at the insertion section.. Under such operational condition, a shorter objective optical system reduces the length from the front end to the bending portion of the endoscope. This dimensional reduction expands the observable area within saccular organs. Furthermore, the objective optical system of the endoscope must satisfy dual requirements: minimal size enabling compact distal architecture; and wide-angle optical characteristics with a field of view greater than or equal to 120 degrees. This substantial field angle introduces significant challenges: difficulty in compensating off-axis aberration at peripheral fields; and heightened sensitivity to surface figure errors and positional tolerances of the optical elements.

### SUMMARY OF THE DISCLOSURE

An object of the present disclosure is to provide an endoscope objective lens that alleviates aberration correction difficulties in existing endoscope lenses.

A further object of the present disclosure is to provide an endoscope incorporating said endoscope objective lens.

According to a first aspect of the present disclosure, an endoscope objective lens provided in some embodiments includes an objective optical system that comprises:
a first lens having a negative optical power, wherein a portion of an image side surface of the first lens close to an optical axis and away from an edge of the first lens is concave;
a second lens, having a positive optical power;
an aperture stop;
a third lens, having a positive optical power;
a fourth lens, having a negative optical power, wherein a portion of an object side surface of the fourth lens close to the optical axis and away from an edge of the fourth lens is concave;
wherein the first lens, the second lens, the aperture stop, the third lens and the fourth lens are arranged in sequence from an image side to an object side along the optical axis;
a chief ray height h11 on an object side surface of the first lens and a chief ray height h42 on an image side surface of the fourth lens satisfy: 0.65 ≤ h11/h42 ≤ 1.35, and/or an effective optical diameter D11 of the object side surface of the first lens and an effective optical diameter D42 of the image side surface of the fourth lens satisfy: 0.75 ≤ D11/D42 ≤ 1.25.

Further, in some embodiments, the chief ray height h11 on the object side surface of the first lens and the chief ray height h42 on the image side surface of the fourth lens satisfy: 0.8≤h11/h42≤1.2.

Further, in some embodiments, the effective optical diameter D11 of the object side surface of the first lens and the effective optical diameter D42 of the image side surface of the fourth lens satisfy: 0.85 ≤ D11/D42 ≤ 1.15.

Further, in some embodiments, the effective optical diameter D11 of the object side surface of the first lens and an imaging height imgH of the objective optical system satisfy: 0.8 ≤ D11/imgH ≤ 1.42.

Further, in some embodiments, the effective optical diameter D11 of the object side surface of the first lens and the imaging height imgH of the objective optical system satisfy: 1.00 ≤ D11/imgH ≤ 1.40.

Further, in some embodiments, chief ray heights at a marginal field of view on two surfaces of the first lens are h11 and h12 respectively, an average of h11 and h12 is H1, a focal length of the first lens is f1, chief ray heights at a marginal field of view on two surfaces of the fourth lens are h41 and h42 respectively, an average of h41 and h42 is H4, a focal length of the fourth lens is f4, whereby the objective optical system satisfies: 1 ≤ (H1/f1)/(H4/f4) ≤ 2.

Further, in some embodiments, a combined focal length F23 of the second lens and the third lens, when optically treated as a lens group, and a focal length f of the objective optical system satisfy: 0.7 ≤ F23/f ≤ 1.2.

Further, in some embodiments, an object side surface of the second lens is convex; and an image side surface of the third lens is convex.

Further, in some embodiments, a number of lenses with optical power in the objective optical system is four.

According to a second aspect of the present disclosure, an endoscope provided in some embodiments includes the endoscope objective lens according to any embodiment of the first aspect.

According to the above-mentioned endoscope objective lens, in the optical system of the endoscope objective lens, the first lens, the second lens, the aperture stop, the third lens and the fourth lens are arranged in sequence from the image side to the object side along the optical axis. The first lens and the fourth lens have negative optical power. The portion of the image side surface of the first lens close to the optical axis and far from the edge of the first lens is concave. The portion of the object side surface of the fourth lens close to the optical axis and far from the edge of the fourth lens is concave. The second lens and the third lens have positive optical power. The chief ray height h11 on the object side surface of the first lens and the chief ray height h42 on the image side surface of the fourth lens satisfy: 0.65 ≤ h11/h42 ≤ 1.35, and/or the effective optical diameter D11 of the object side surface of the first lens and the effective optical diameter D42 of the image side surface of the fourth lens satisfy: 0.75 ≤ D11/D42 ≤ 1.25. The aperture stop is disposed between the second lens and the third lens in the objective optical system, and the optical powers of lenses at symmetric positions on either side of the aperture stop exhibit symmetry in sign. In addition, the object side surface of the first lens and the image side surface of the fourth lens are approximately symmetric in shape. The positions of the rays passing through the optical surfaces of the two approximately symmetric lenses, especially the first lens and the fourth lens, are approximately symmetric. The above-mentioned approximately symmetric features can make the entire optical system have a certain degree of symmetry. By taking advantage of the characteristic that the signs of the partial aberrations produced by the approximately symmetric surfaces in the optical system are opposite, some aberrations can be mutually offset, thereby reducing aberration correction difficulty and improving imaging quality.

### BRIEF DESCRIPTION OF THE DRAWINGS

FIG. 1 is a structural schematic diagram of an objective optical system according to a first embodiment;
FIG. 2 is an imaging diffraction MTF curve diagram for the objective optical system of FIG. 1;
FIG. 3 is a structural schematic diagram of an objective optical system according to a second embodiment;
FIG. 4 is an imaging diffraction MTF curve diagram for the objective optical system of FIG. 3;
FIG. 5 is a structural schematic diagram of an objective optical system according to a third embodiment;
FIG. 6 is an imaging diffraction MTF curve diagram for the objective optical system of FIG. 5;
FIG. 7 is a structural schematic diagram of an objective optical system according to a fourth embodiment;
FIG. 8 is an imaging diffraction MTF curve diagram for the objective optical system of FIG. 7;
FIG. 9 is a structural schematic diagram of the objective optical system according to a fifth embodiment;
FIG. 10 is an imaging diffraction MTF curve diagram for the objective optical system of FIG. 9;
FIG. 11 is a structural schematic diagram of an objective optical system according to a sixth embodiment;
FIG. 12 is an imaging diffraction MTF curve diagram for the objective optical system of FIG. 11;
FIG. 13 is a structural schematic diagram of an objective optical system according to a seventh embodiment;
FIG. 14 is an imaging diffraction MTF curve diagram for the objective optical system of FIG. 13;
FIG. 15 is a structural schematic diagram of an objective optical system according to an eighth embodiment;
FIG. 16 is an imaging diffraction MTF curve diagram for the objective optical system of FIG. 15;
FIG. 17 is an structural schematic diagram of an objective optical system according to a ninth embodiment; and
FIG. 18 is an imaging diffraction MTF curve diagram for the objective optical system of FIG. 17.

### List of reference numerals:

- 1: first lens;
- 11: object side surface;
- 12: image side surface;
- 2: second lens;
- 21: object side surface;
- 22: image side surface;
- 3: third lens;
- 31: object side surface;
- 32: image side surface;
- 4: fourth lens;
- 41: object side surface;
- 42: image side surface;
- 5: aperture stop;
- 6: optical filter;
- 61: object side surface;
- 62: image side surface;
- 7: image sensor glass;
- 71: object side surface;
- 72: image side surface.

### DETAILED DESCRIPTION

Specific embodiments of the present disclosure will now be described in detail with reference to the accompanying drawings. Similar or related components in different embodiments are labeled with associated reference numerals. The following embodiments include detailed descriptions to facilitate understanding of the present disclosure. However, those skilled in the art will readily recognize that certain features may be omitted under specific circumstances or substituted by other components, materials, or methods. In some instances, certain operations related to the present disclosure are not explicitly described or illustrated herein. This intentional exclusion is intentional to avoid obscuring the core technical solutions of the present disclosure. For those skilled in the art, a complete understanding of these operations can be attained through the descriptions provided in this specification and general technical knowledge in the art.

Additionally, the features, operations, or characteristics described in the specification may be combined in any suitable manner to form various embodiments. Similarly, steps or actions in the method descriptions may be reordered or modified in ways that would be obvious to those skilled in the art. Therefore, the sequences presented in the specification and drawings are intended solely to clarify the description of specific embodiments and do not imply mandatory orderings, unless explicitly stated that a particular sequence is required.

The numerical designations assigned to components in this specification, such as 'first,' 'second,' or similar ordinal terms, serve solely to distinguish described objects and carry no inherent sequential or technical implications. Furthermore, the terms 'connected' and 'coupled' as used herein encompass both direct and indirect connection (coupling), unless explicitly stated otherwise.

The field of view of an endoscope objective lens is generally greater than or equal to 120 degrees, so the objective optical system of an endoscope is a wide-angle objective optical system. Due to the large field of view, the off-axis aberrations caused by the edge rays of the endoscope objective lens are particularly difficult to correct. This application addresses this challenge by providing an endoscope objective optical system featuring an approximately symmetric structure. The approximate symmetry of the optical surfaces and the ray paths traversing them enables mutual compensation of certain aberrations. This configuration is therefore beneficial for aberration correction, with particular pronounced effects on correcting coma, distortion, and lateral chromatic aberration. An exemplary structure for the endoscope objective lens is described in detail below.

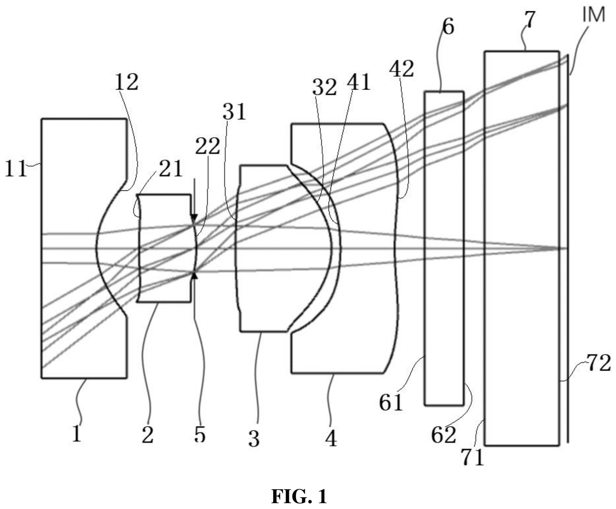

In some embodiments, as shown in FIGS. 1 to 16, the endoscope objective lens comprises an objective optical system that including a first lens 1, a second lens 2, an aperture stop, a third lens 3, and a fourth lens 4. The first lens 1, the second lens 2, the aperture stop, the third lens 3, and the fourth lens 4 are sequentially arranged along the optical axis of the objective optical system from the image side to the object side.

In some embodiments, to satisfy low-cost manufacturing constraints, the objective optical system comprises four lenses with optical power, with the aperture stop positioned at the middle position of the four lenses, thereby achieving approximate structural symmetry.

In some embodiments, for a wide-angle objective lens, to increase the chief ray angle at the field edge, the first lens 1 has a negative optical power. Specifically, the portion of the image side surface 12 of the first lens 1 close to the optical axis of the objective optical system and away from the edge of the first lens 1 is concave.

The first lens 1 may adopt any feasible lens shape. For example, in an embodiment, the first lens 1 adopts a meniscus lens. In another embodiment, the first lens 1 is a plano-concave lens, with the object side surface 11 being planar and the image side surface 12 being concave.

In some embodiments, based on a similar symmetric structure, the fourth lens 4 and the first lens 1 of the objective optical system are approximately symmetric, and the fourth lens 4 also has a negative optical power. A portion of the object side surface 41 of the fourth lens 4 close to the optical axis of the objective optical system and away from the edge of the fourth lens 4 is concave.

Specifically, in some embodiments, the fourth lens 4 is a meniscus lens, with the object side surface 41 being concave.

In some embodiments, the first lens 1 and the fourth lens 4 both have negative optical power; and the second lens 2 and the third lens 3 both have positive optical power. This makes the entire objective optical system have a positive optical power for imaging. In an embodiment, the image side surface 32 of the third lens 3 is convex.

In some embodiments, the objective optical system satisfies at least one of the following two conditions:
Condition 1: The chief ray height h11 on the object side surface 11 of the first lens 1 and the chief ray height h42 on the image side surface 42 of the fourth lens 4 satisfy: 0.65 ≤ h11/h42 ≤ 1.35.
Condition 2: The effective optical diameter D11 of the object side surface 11 of the first lens 1 and the effective optical diameter D42 of the image side surface 42 of the fourth lens 4 satisfy: 0.75 ≤ D11/D42 ≤ 1.25.

The objective optical system satisfying condition 1 and/or condition 2 makes the heights at which the rays pass through the first lens 1 and the fourth lens 4 approximately symmetric, which is beneficial for aberration correction and also helps to reduce the sensitivity of the optical surfaces to tolerances. The chief ray height at the marginal field of view on the object side surface 11 of the first lens 1 is close to the chief ray height at the marginal field of view on the image side surface 42 of the fourth lens 4, which can make the heights at which the rays pass approximately symmetric. The effective optical diameter of the object side surface 11 of the first lens 1 is close to the effective optical diameter of the image side surface 42 of the fourth lens 4, which may also make the ray heights when passing through these lenses approximately symmetric.

In other words, in the objective optical system of this application, in addition to the approximately symmetric structure of the first lens 1 and the fourth lens 4, the ray heights when passing through the approximately symmetric first lens 1 and fourth lens 4 are also approximately symmetric.

It should be noted that in this application, the object side of the objective optical system may be understood as the side of the objective optical system facing an object to be photographed, such as a lesion area, and the image side of the objective optical system may be understood as the side where the imaging surface of the objective optical system is located. Similarly, the image side surface of the lens is the side of the lens facing the imaging surface of the objective optical system, and the object side surface is the side of the lens facing the object to be photographed.

In some embodiments, to further optimize the parameters, the chief ray height h11 on the object side surface 11 of the first lens 1 and the chief ray height h42 on the image side surface 42 of the fourth lens 4 satisfy: 0.8 ≤ h11/h42 ≤ 1.2. In other embodiments, h11/h42 may satisfy 0.65 ≤ h11/h42 ≤ 0.85, or 1.2 ≤ h11/h42 ≤ 1.35.

In some embodiments, to further optimize the parameters, the effective optical diameter D11 of the object side surface 11 of the first lens 1 and the effective optical diameter D42 of the image side surface 42 of the fourth lens 4 satisfy: 0.85 ≤ D11/D42 ≤ 1.15. In other embodiments, D11/D42 may satisfy: 0.75 ≤ D11/D42 ≤ 0.85, or 1.15 ≤ D11/D42 ≤ 1.25.

Furthermore, the distal tip of the endoscope lens includes components such as the endoscope objective, imaging module, lighting element, instrument channel, water and gas channel, and structural support element. Generally, the size of the distal tip of the endoscope lens and the size of the instrument channel are primarily determined by clinical needs. The imaging module includes an image sensor, and the size of the image sensor is determined by the clinical requirements for image clarity and pixel count, as well as the remaining space at the distal tip. The larger the size of the image sensor, the better the image quality usually is. Therefore, when designing an endoscope, the best practice is that the size of the entire imaging module is determined by the maximum size of the selected image sensor and its associated circuit board, that is, the diameter of the endoscope objective should be less than or equal to the size of the image sensor and its associated circuit board. Under the conditions that the image sensor packaging process and the objective optical component process are determined, this means that there is a certain relationship between the diameter of the endoscope objective optical system and the effective photosensitive surface (i.e., image plane size) of the image sensor.

The ratio of the effective optical diameter D11 of the object side surface 11 of the first lens 1 to the imaging height imgH of the optical system is D11/imgH. When D11/imgH is too small, the effective optical diameter of the first lens 1 is too small, and the imaging beam is overly concentrated at the first lens 1. Since the endoscope is used in the human body, the optical surface of the objective (i.e., the object side surface 11 of the first lens 1) is prone to being contaminated by foreign objects. Objective contaminants may affect the image quality. When the imaging beam is overly concentrated at the first lens 1, a larger portion of the imaging beam is affected by contaminant particles, and the impact of the contaminants on the image quality is greater. At the same time, considering the constraints of h11/h42 and/or D11/D42, if D11/imgH is too small, the maximum effective optical diameter of the fourth lens 4 is also too small, and at this time, the light beam is overly concentrated at the image side surface 42 of the fourth lens 4, increasing the tolerance sensitivity and demanding tighter tolerances for surface shape accuracy and assembly precision of the fourth lens 4. If D11/imgH is too large, the effective optical diameter of the lens is too large, and the final diameter of the objective will be larger than the package dimensions of the image sensor, resulting in a larger module size and being unfavorable for minimizing the diameter of the objective. In an embodiment, the relationship between the effective optical diameter D11 of the object side surface 11 of the first lens 1 in the objective optical system and the imaging height imgH (i.e., image height) of the optical system satisfies the following condition: 0.8 ≤ D11/imgH ≤ 1.42. When D11/imgH is within this range, the objective optical system is less affected by contaminants and has a lower tolerance sensitivity. Further, in an embodiment, 1.00 ≤ D11/imgH ≤ 1.40. In other embodiments, depending on the application requirements, D11/imgH may be less than 0.8 or greater than 1.42 without considering the above factors.

Further, in some embodiments, to further optimize the objective lens size and image quality, the chief ray heights of the marginal field at the two surfaces of the first lens 1 are h11 and h12, respectively, and the average value of h11 and h12 is H1. The focal length of the first lens 1 is f1. The chief ray heights of the marginal field at the two surfaces of the fourth lens 4 are h41 and h42, respectively, and the average value of h41 and h42 is H4. The focal length of the fourth lens 4 is f4. Then, the objective optical system satisfies the following condition: 1 ≤ (H1/f1) / (H4/f4) ≤ 2. H1/f1 and H4/f4, to some extent, represent the refractive power of the lenses. When the difference between the two is small, the light rays in the objective lens are more symmetric, which is beneficial for achieving wide-angle performance, shortening the objective lens length, and providing higher relative illumination at the marginal field, making it easier to correct distortion. In other embodiments, (H1/f1) / (H4/f4) may also be less than 1 or greater than 2.

Further, the second lens 2 and the third lens 3 are positive optical power lenses. The main function of the second lens 2 and the third lens 3 is to converge light rays. When the optical power of the second lens 2 and the third lens 3 is strong, it is beneficial for light convergence, thereby reducing the length of the objective lens and enhancing the wide-angle performance. Therefore, in some embodiments, the combined focal length of the second lens 2 and the third lens 3 as a group of lenses is F23, and the focal length of the objective lens is f. The objective optical system satisfies the following condition: 0.7 ≤ F23/f ≤ 1.2. This allows for a shorter objective lens length. If F23/f is too large, it may lead to an excessively long objective lens. If F23/f is too small, it makes aberration correction difficult, resulting in degraded imaging quality. In other embodiments, when conditions permit, F23/f may be less than 0.7 or greater than 1.2.

Further, in some embodiments, the object side surface 21 of the second lens 2 is convex; and the image side surface 32 of the third lens 3 is convex. This is more conducive to the symmetry of the objective optical system. In some embodiments, the second lens 2 and the third lens 3 may be biconvex lenses, where both the object side surface and the image side surface of the lens are convex. In other embodiments, one of the object side surface 21 of the second lens 2 and the image side surface 32 of the third lens 3 is concave, or both are concave. In other embodiments, one of the object side surface 21 of the second lens 2 and the image side surface 32 of the third lens 3 is convex.

Considering that if the thickness of the first lens 1 is too thick, it may increase the length of the objective lens and reduce the effectiveness of the approximate symmetry of the objective lens structure, in some embodiments, the objective optical system satisfies the following condition: D11/T1 ≥ 3.2, where D11 is the effective optical diameter of the object side surface 11 of the first lens 1, and T1 is the center thickness of the first lens 1.

Based on the description of the above embodiments, the following describes in detail specific embodiments with reference to examples.

### First embodiment (Embodiment 1):

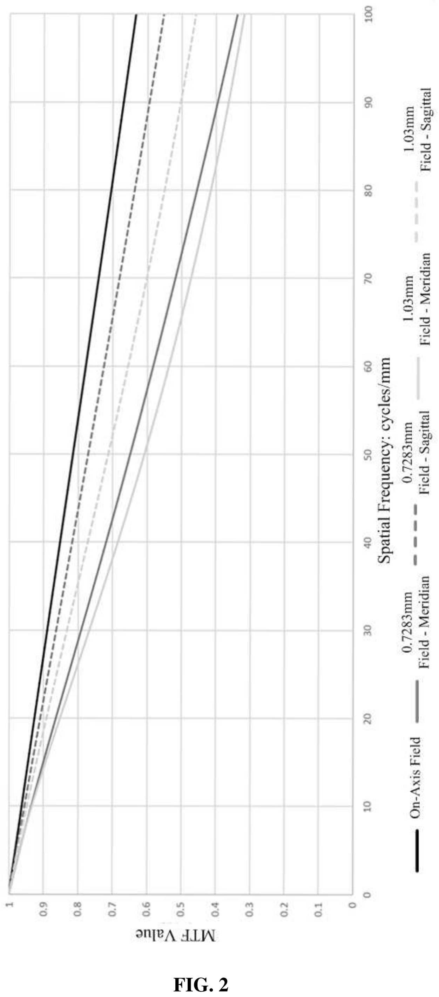

As shown in FIGS. 1 and 2, the left side is the object side and the right side is the image side, with IM denoting the image plane. From the object side to the image plane, the optical components are arranged in the following order: the first lens 1, the second lens 2, the aperture stop 5, the third lens 3, the fourth lens 4, the optical filter 6, and the image sensor glass 7 (e.g., a cover glass for the image sensor).

The optical filter 6 has an object side surface 61 and an image side surface 62. The image sensor glass 7 has an object side surface 71 and an image side surface 72. In some embodiments, the optical filter 6 and the image sensor glass 7 are not necessary, and their use in the objective optical system may be selected based on requirements. Referring to FIG. 1, the first lens 1 has a negative optical power, with its object side surface 11 (i.e., left side) being a planar surface, and image side surface 12 (i.e., right side) being a concave surface. The second lens 2 has a positive optical power, with its object side surface 21 being a W-shaped surface, and image side surface 22 being a convex surface. The third lens 3 has a positive optical power and is a biconvex lens, with both object side surface 31 and image side surface 32 being convex surfaces. The fourth lens 4 has a negative optical power, with its object side surface 41 being a concave surface, and image side surface 42 being a W-shaped surface.

**Table 1 Construction Data of the Objective Optical System in Embodiment 1**

| f=0.750 | FOV=140° | F/4.95 | Image Height imgH=1.03 | | | | Total Track Length | |
|---|---|---|---|---|---|---|---|---|
| | | | TTL=2.898 | | | | | |
| Surface Number | Name | Surface Profile | Radius of Curvature | Thic knes s | Refracti ve Index | Abbe Numbe r | Effective Diameter | Focal Length |
| Object Plane | | | Infinite | 10.0 000 | | | 57.44 | |
| Object Side Surface 11 | First Lens 1 | Standard Spherical Surface | Infinite | 0.29 40 | 1.5445 | 55.99 | 1.28 | -0.681 3 |
| Image Side Surface 12 | | Even Aspheric Surface | 0.3710 | 0.22 71 | | | 0.63 | |
| Object Side | Second | Even | 1.5834 | 0.30 | 1.5445 | 55.99 | 0.47 | 0.8329 |
| Surface 21 | Lens 2 | Aspheric Surface | | 62 | | | | |
| Image Side Surface 22 | | Even Aspheric Surface | -0.5922 | -0.01 01 | | | 0.26 | |
| Aperture Plane | Aperture Stop 5 | Standard Spherical Surface | Infinite | 0.21 92 | | | 0.25 | |
| Object Side Surface 31 | Third Lens 3 | Even Aspheric Surface | 3.4502 | 0.51 25 | 1.5445 | 55.99 | 0.58 | 0.6207 |
| Image Side Surface 32 | | Even Aspheric Surface | -0.3551 | 0.04 75 | | | 0.78 | |
| Object Side Surface 41 | Fourth Lens 4 | Even Aspheric Surface | -0.8314 | 0.28 95 | 1.651 | 21.51 | 0.80 | -0.818 |
| Image Side Surface 42 | | Even Aspheric Surface | 1.6846 | 0.15 91 | | | 1.23 | |
| Object Side Surface 61 | Optical Filter 6 | Standard Spherical Surface | Infinite | 0.21 00 | 1.5233 | 54.52 | 1.43 | |
| Image Side Surface 62 | | Standard Spherical Surface | Infinite | 0.10 98 | | | 1.57 | |
| Object Side Surface 71 | Image Sensor Glass 7 | Standard Spherical Surface | Infinite | 0.40 00 | 1.5168 | 64.2 | 1.69 | |
| Image Side Surface 72 | | Standard Spherical Surface | Infinite | 0.04 50 | | | 2.00 | |
| IM | Image Plane | Standard Spherical Surface | Infinite | | | | 2.07 | |

The imaging quality in this embodiment is excellent, with MTF data provided in FIG. 2. Table 1 provides the following parameters for each element in Embodiment 1: radius of curvature, thickness, refractive index, Abbe number, effective optical diameter, focal length f of the objective lens, field of view (FOV), F-number, and total track length (TTL). Table 2 provides the aspheric data of all elements.

**Table 2 Aspheric Data of Aspheric Surfaces in Embodiment 1**

| Aspheric Coefficient | | | | | | | | |
|---|---|---|---|---|---|---|---|---|
| Surface Number | Conic Constant | A4 | A6 | A8 | A10 | A12 | A14 | A16 |
| Object Plane | 0.00E+00 | 0.0000E +00 | 0.0000E +00 | 0.0000E +00 | 0.0000E +00 | 0.0000E +00 | 0.0000E +00 | 0.0000E +00 |
| Object Side Surface 11 | 0.00E+00 | 0.0000E +00 | 0.0000E +00 | 0.0000E +00 | 0.0000E +00 | 0.0000E +00 | 0.0000E +00 | 0.0000E +00 |
| Image Side Surface 12 | -1.72E+0 0 | 9.6206E -01 | 7.8239E +00 | -1.2147 E+02 | -6.0243 E+02 | 7.4047E +03 | 8.0838E +04 | -6.8264 E+05 |
| Object Side Surface 21 | -1.00E+02 | -2.3872E+00 | 2.5672E+01 | -1.5410E+03 | 1.2296E+04 | 2.7215E+04 | 1.6627E+06 | -2.7656E+07 |
| Image Side Surface 22 | 2.77E+00 | 3.9380E +00 | -2.6881 E+02 | -2.3568 E+03 | 9.7929E +05 | -5.7178 E+06 | -1.9808 E+09 | 4.4413E +10 |
| Aperture Plane | 0.00E+00 | 0.0000E +00 | 0.0000E +00 | 0.0000E +00 | 0.0000E +00 | 0.0000E +00 | 0.0000E +00 | 0.0000E +00 |
| Object Side Surface 31 | 1.54E+01 | -3.4267 E-01 | 2.1059E +01 | -7.3756 E+02 | 8.9405E +03 | -3.2309 E+04 | -1.0312 E+05 | 1.0403E +06 |
| Image Side Surface 32 | -1.14E+0 0 | 3.43E+0 0 | -5.67E+ 01 | 3.53E+0 2 | -1.04E+ 03 | 2.92E+0 3 | -1.19E+ 04 | 3.10E+0 4 |
| Object Side Surface 41 | 1.88E+00 | 1.83E+0 0 | -5.66E+ 01 | 1.61E+0 2 | 1.53E+0 3 | -1.21E+ 04 | 3.53E+0 4 | -5.12E+ 04 |
| Image Side Surface 42 | -8.27E+0 1 | 6.4746E -01 | -1.8609 E+01 | 1.1230E +02 | -3.4970 E+02 | 5.9650E +02 | -5.2845 E+02 | 1.8603E +02 |
| Object Side Surface 61 | 0.00E+00 | 0.0000E +00 | 0.0000E +00 | 0.0000E +00 | 0.0000E +00 | 0.0000E +00 | 0.0000E +00 | 0.0000E +00 |
| Image Side Surface 62 | 0.00E+00 | 0.0000E +00 | 0.0000E +00 | 0.0000E +00 | 0.0000E +00 | 0.0000E +00 | 0.0000E +00 | 0.0000E +00 |
| Object Side Surface 71 | 0.00E+00 | 0.0000E +00 | 0.0000E +00 | 0.0000E +00 | 0.0000E +00 | 0.0000E +00 | 0.0000E +00 | 0.0000E +00 |
| Image Side Surface 72 | 0.00E+00 | 0.0000E +00 | 0.0000E +00 | 0.0000E +00 | 0.0000E +00 | 0.0000E +00 | 0.0000E +00 | 0.0000E +00 |
| Image Plane | 0.00E+00 | 0.0000E +00 | 0.0000E +00 | 0.0000E +00 | 0.0000E +00 | 0.0000E +00 | 0.0000E +00 | 0.0000E +00 |

### Second embodiment (Embodiment 2):

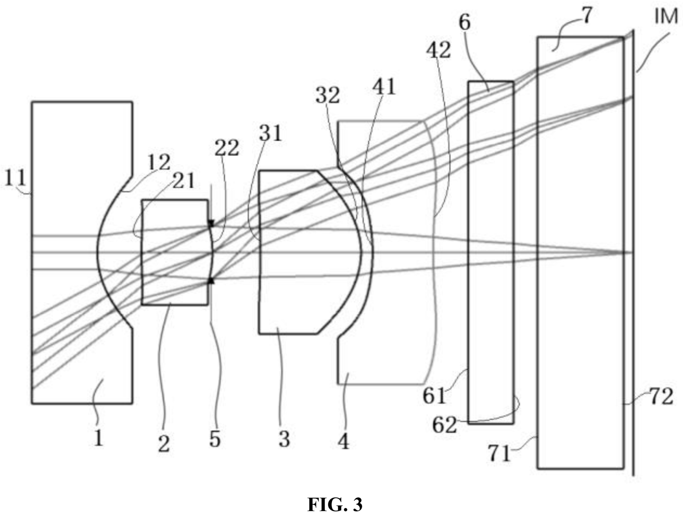

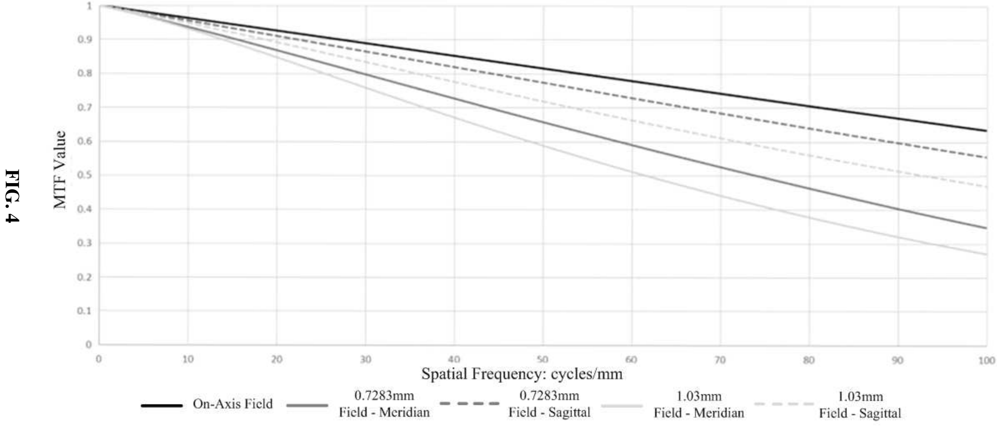

As shown in FIGS. 3 and 4, the left side is the object side and the right side is the image side, with IM denoting the image plane. From the object side to the image plane, the optical components are arranged in the following order: the first lens 1, the second lens 2, the aperture stop 5, the third lens 3, the fourth lens 4, the optical filter 6, and the image sensor glass 7. In some embodiments, the optical filter 6 and the image sensor glass 7 are not necessary, and their use in the objective optical system may be selected based on requirements. Referring to FIG. 3, the first lens 1 has a negative optical power, with its object side surface 11 (the left side) being a planar surface, and image side surface 12 (the right side) being a concave surface. The second lens 2 has a positive optical power, with its object side surface 21 being a W-shaped surface, and image side surface 22 being a convex surface. The third lens 3 has a positive optical power, with its object side surface 31 being a concave surface, and image side surface 32 being a convex surface. The fourth lens 4 has a negative optical power, with its object side surface 41 being a concave surface, and image side surface 42 being W-shaped surface.

**Table 3 Construction Data of the Objective Optical System in Embodiment 2**

| f=0.758 | FOV=140° | F/4.968 | Image Height imgH=1.03 | | Total Track Length | | | |
|---|---|---|---|---|---|---|---|---|
| | | | TTL=2.782 | | | | | |
| Surface Number | Name | Surface Profile | Radius of Curvature | Thick ness | Refra ctive Index | Abb e Num ber | Effec tive Diam eter | Foca 1 Len gth |
| Object Plane | | | Infinite | 10.0000 | | | 57.49 | |
| Object Side Surface 11 | First Lens 1 | Standard Spherical Surface | Infinite | 0.302 8 | 1.544 5 | 55.9 9 | 1.27 | -0.6 978 |
| Image Side Surface 12 | | Even Aspheric Surface | 0.3800 | 0.204 4 | | | 0.61 | |
| Object Side Surface 21 | Second Lens 2 | Even Aspheric Surface | 1.8054 | 0.328 1 | 1.544 5 | 55.9 9 | 0.49 | 0.75 54 |
| Image Side Surface 22 | | Even Aspheric Surface | -0.4986 | 0.000 0 | | | 0.29 | |
| Aperture Plane | Aperture Stop 5 | Standard Spherical Surface | Infinite | 0.221 3 | | | 0.24 | |
| Object Side Surface 31 | Third Lens3 | Even Aspheric Surface | -5.0000 | 0.466 1 | 1.544 5 | 55.9 9 | 0.55 | 0.64 10 |
| Image Side Surface 32 | | Even Aspheric Surface | -0.3370 | 0.053 4 | | | 0.76 | |
| Object Side Surface 41 | Fourth Lens 4 | Even Aspheric Surface | -0.7945 | 0.279 9 | 1.651 | 21.5 1 | 0.79 | -0.8 785 |
| Image Side Surface 42 | | Even Aspheric Surface | 2.3241 | 0.162 1 | | | 1.22 | |
| Object Side Surface 61 | Optical Filter 6 | Standard Spherical Surface | Infinite | 0.210 0 | 1.523 3 | 54.5 2 | 1.45 | |
| Image Side Surface 62 | | Standard Spherical Surface | Infinite | 0.109 3 | | | 1.59 | |
| Object Side Surface 71 | Image Sensor Glass 7 | Standard Spherical Surface | Infinite | 0.400 0 | 1.516 8 | 64.2 | 1.71 | |
| Image Side Surface 72 | | Standard Spherical Surface | Infinite | 0.045 0 | | | 2.00 | |
| IM | Image Plane | Standard Spherical Surface | Infinite | | | | 2.07 | |

**Table 4 Aspheric Data of Aspheric Surfaces in Embodiment 2**

| Aspheric Coefficient | | | | | | | | |
|---|---|---|---|---|---|---|---|---|
| Surface Number | Conic Constant | A4 | A6 | A8 | A10 | A12 | A14 | A16 |
| Object Plane | 0.0000E+ 00 | 0.0000E +00 | 0.0000E +00 | 0.0000E +00 | 0.0000E +00 | 0.0000E +00 | 0.0000E +00 | 0.0000E +00 |
| Object Side Surface 11 | 0.0000E+ 00 | 0.0000E +00 | 0.0000E +00 | 0.0000E +00 | 0.0000E +00 | 0.0000E +00 | 0.0000E +00 | 0.0000E +00 |
| Image Side Surface 12 | -1.6127E +00 | 1.2394E +00 | 7.0415E +00 | -6.1781 E+01 | -6.9056 E+02 | 2.8530E +03 | 5.8832E +04 | -3.6758 E+05 |
| Object Side Surface 21 | -5.5165E +01 | -2.8182 E+00 | 8.7161E +00 | -9.5029 E+02 | 1.2443E +04 | -9.9787 E+04 | 2.8638E +04 | 5.2854E +06 |
| Image Side Surface 22 | -5.4764E-01 | 1.5171E +00 | -2.1171 E+02 | 5.3014E +03 | 1.8964E +05 | -6.2053 E+06 | -4.6962 E+08 | 1.5063E +10 |
| Aperture Plane | 0.0000E+00 | 0.0000E+00 | 0.0000E+00 | 0.0000E+00 | 0.0000E+00 | 0.0000E+00 | 0.0000E+00 | 0.0000E+00 |
| Object Side Surface 31 | 5.7860E+ 00 | -5.0174 E-01 | 2.1860E +01 | -5.3828 E+02 | 5.8520E +03 | -1.8891 E+04 | 8.3400E +04 | -1.4279 E+05 |
| Image Side Surface 32 | -9.9516E-01 | 3.45E+0 0 | -5.16E+ 01 | 3.19E+0 2 | -1.08E+ 03 | 2.98E+0 3 | -1.13E+ 04 | 6.12E+0 4 |
| Object Side Surface 41 | 1.7605E+ 00 | 2.92E+0 0 | -5.94E+ 01 | 1.72E+0 2 | 1.45E+0 3 | -1.29E+ 04 | 3.74E+0 4 | -4.02E+ 04 |
| Image Side Surface 42 | -6.6080E +01 | 6.6577E -01 | -1.9072 E+01 | 1. 1508E +02 | -3.6189 E+02 | 6.1888E +02 | -5.4297 E+02 | 1.8559E +02 |
| Object Side Surface 61 | 0.0000E+ 00 | 0.0000E +00 | 0.0000E +00 | 0.0000E +00 | 0.0000E +00 | 0.0000E +00 | 0.0000E +00 | 0.0000E +00 |
| Image Side Surface 62 | 0.0000E+ 00 | 0.0000E +00 | 0.0000E +00 | 0.0000E +00 | 0.0000E +00 | 0.0000E +00 | 0.0000E +00 | 0.0000E +00 |
| Object Side Surface 71 | 0.0000E+ 00 | 0.0000E +00 | 0.0000E +00 | 0.0000E +00 | 0.0000E +00 | 0.0000E +00 | 0.0000E +00 | 0.0000E +00 |
| Image Side Surface 72 | 0.0000E+ 00 | 0.0000E +00 | 0.0000E +00 | 0.0000E +00 | 0.0000E +00 | 0.0000E +00 | 0.0000E +00 | 0.0000E +00 |
| Image Plane | 0.0000E+ 00 | 0.0000E +00 | 0.0000E +00 | 0.0000E +00 | 0.0000E +00 | 0.0000E +00 | 0.0000E +00 | 0.0000E +00 |

The imaging quality in this embodiment is excellent, with MTF data provided in FIG. 4. Table 3 provides the following parameters for each element in Embodiment 2: radius of curvature, thickness, refractive index, Abbe number, effective optical diameter, focal length f of the objective lens, field of view (FOV), F-number, and total track length (TTL). Table 4 provides the aspheric data of all elements.

### Third Embodiment (Embodiment 3):

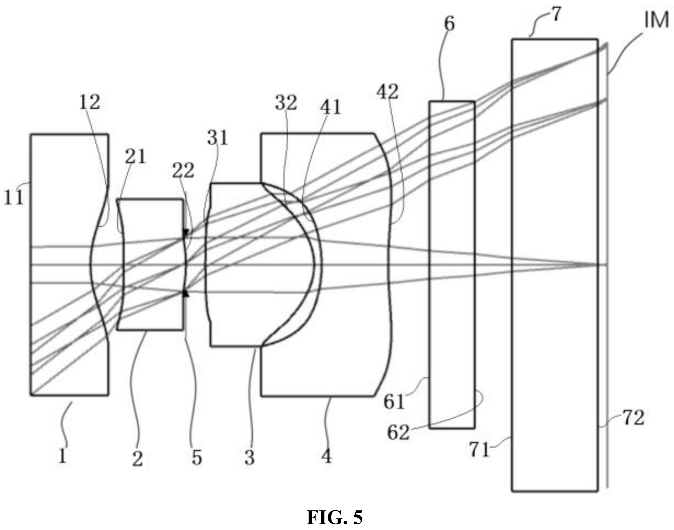

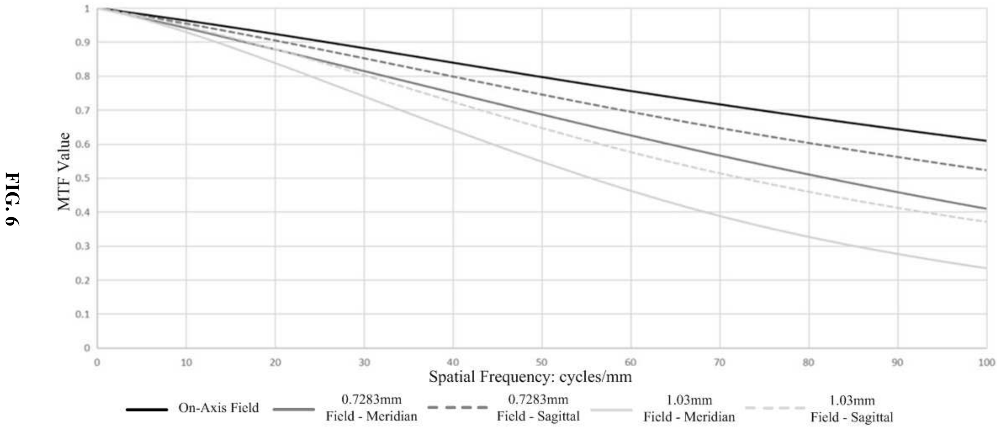

As shown in FIGS. 5 and 6, the left side is the object side and the right side is the image side, with IM denoting the image plane. From the object side to the image plane, the optical components are arranged in the following order: the first lens 1, the second lens 2, the aperture stop 5, the third lens 3, the fourth lens 4, the optical filter 6, and the image sensor glass 7. In some embodiments, the optical filter 6 and the image sensor glass 7 are not necessary, and their use in the objective optical system may be selected based on requirements.

**Table 5 Construction Data of the Objective Optical System in Embodiment 3**

| f=0.826 | FOV=140° | F/4.6246 | Image Height imgH=1.03 | | | Total Track Length | | |
|---|---|---|---|---|---|---|---|---|
| | | | TTL=2.693 | | | | | |
| Surface Number | Name | Surface Profile | Radius of Curvatu re | Thickne ss | Refracti ve Index | Abbe Numb er | Effecti ve Diamet er | Focal Lengt h |
| Object Plane | | | Infinite | 10.0000 | | | 57.24 | |
| Object Side Surface 11 | First Lens 1 | Standard Spherica 1 Surface | Infinite | 0.2788 | 1.5445 | 55.99 | 1.22 | -0.81 35 |
| Image Side Surface 12 | | Even Aspheric Surface | 0.4430 | 0.1557 | | | 0.67 | |
| Object Side | Second Lens | Even | -11.018 | 0.2901 | 1.5445 | 55.99 | 0.51 | 1.225 |
| Surface 21 | 2 | Aspheric Surface | 4 | | | | | 5 |
| Image Side Surface 22 | | Even Aspheric Surface | -0.6350 | -0.0103 | | | 0.25 | |
| Aperture Plane | Aperture Stop 5 | Standard Spherica 1 Surface | Infinite | 0.1015 | | | 0.25 | |
| Object Side Surface 31 | Third Lens 3 | Even Aspheric Surface | 6.4504 | 0.5082 | 1.5445 | 55.99 | 0.44 | 0.464 9 |
| Image Side Surface 32 | | Even Aspheric Surface | -0.2562 | 0.0346 | | | 0.66 | |
| Object Side Surface 41 | Fourth Lens 4 | Even Aspheric Surface | -0.5830 | 0.3111 | 1.651 | 21.51 | 0.66 | -0.62 55 |
| Image Side Surface 42 | | Even Aspheric Surface | 1.6344 | 0.1930 | | | 1.12 | |
| Object Side Surface 61 | Optical Filter 6 | Standard Spherica 1 Surface | Infinite | 0.2100 | 1.5233 | 54.52 | 1.38 | |
| Image Side Surface 62 | | Standard Spherica 1 Surface | Infinite | 0.1753 | | | 1.52 | |
| Object Side Surface 71 | Image Sensor Glass 7 | Standard Spherica 1 Surface | Infinite | 0.4000 | 1.5168 | 64.2 | 1.72 | |
| Image Side Surface 72 | | Standard Spherica 1 Surface | Infinite | 0.0450 | | | 2.00 | |
| IM | Image Plane | Standard Spherica 1 Surface | Infinite | | | | 2.07 | |

**Table 6 Aspheric Data of Aspheric Surfaces in Embodiment 3**

| Aspheric Coefficient | | | | | | | | |
|---|---|---|---|---|---|---|---|---|
| Surface Number | Conic Constant | A4 | A6 | A8 | A10 | A12 | A14 | A16 |
| Object Plane | 0.00E+00 | 0.0000E +00 | 0.0000E +00 | 0.0000E +00 | 0.0000E +00 | 0.0000E +00 | 0.0000E +00 | 0.0000E +00 |
| Object Side Surface 11 | 0.00E+00 | 0.0000E +00 | 0.0000E +00 | 0.0000E +00 | 0.0000E +00 | 0.0000E +00 | 0.0000E +00 | 0.0000E +00 |
| Image Side Surface 12 | -3.88E+00 | -3.1828E -01 | 5.1996E-01 | -1.7082E +02 | -6.7266E +02 | 8.4951E +03 | 9.9085E +04 | -6.2936E +05 |
| Object Side Surface 21 | 1.00E+02 | -5.8302E +00 | 3.5116E +01 | -9.5530E +02 | 1.3372E +04 | -2.9553E +04 | 1.9862E +05 | -4.0831E +06 |
| Image Side Surface 22 | 1.67E+00 | -2.0202E +00 | 1.8495E +02 | 6.4832E +02 | 8.0469E +05 | -2.5877E +07 | -1.9330E +09 | 6.7561E +10 |
| Aperture Plane | 0.00E+00 | 0.0000E +00 | 0.0000E +00 | 0.0000E +00 | 0.0000E +00 | 0.0000E +00 | 0.0000E +00 | 0.0000E +00 |
| Object Side Surface 31 | 5.39E+01 | 2.3595E +00 | 1.1296E +01 | -5.2846E +02 | 1.0494E +04 | -1.6552E +04 | 2.4935E +04 | -3.2951E +06 |
| Image Side Surface 32 | -1.13E+00 | 3.42E+0 0 | -5.92E+ 01 | 3.03E+0 2 | -8.35E+ 02 | 2.84E+0 3 | -6.68E+ 03 | 1.65E+0 5 |
| Object Side Surface 41 | 1.04E+00 | 2.69E+0 0 | -6.02E+ 01 | 1.44E+0 2 | 1.25E+0 3 | -1.67E+ 04 | 2.79E+0 4 | 1.55E+0 4 |
| Image Side Surface 42 | -1.00E+02 | 5.0185E-01 | -1.8067E +01 | 1. 1255E +02 | -3.6693E +02 | 6.0174E +02 | -4.6749E +02 | 2.0124E +02 |
| Object Side Surface 61 | 0.00E+00 | 0.0000E +00 | 0.0000E +00 | 0.0000E +00 | 0.0000E +00 | 0.0000E +00 | 0.0000E +00 | 0.0000E +00 |
| Image Side Surface 62 | 0.00E+00 | 0.0000E +00 | 0.0000E +00 | 0.0000E +00 | 0.0000E +00 | 0.0000E +00 | 0.0000E +00 | 0.0000E +00 |
| Object Side Surface 71 | 0.00E+00 | 0.0000E +00 | 0.0000E +00 | 0.0000E +00 | 0.0000E +00 | 0.0000E +00 | 0.0000E +00 | 0.0000E +00 |
| Image Side Surface 72 | 0.00E+00 | 0.0000E +00 | 0.0000E +00 | 0.0000E +00 | 0.0000E +00 | 0.0000E +00 | 0.0000E +00 | 0.0000E +00 |
| Image Plane | 0.00E+00 | 0.0000E +00 | 0.0000E +00 | 0.0000E +00 | 0.0000E +00 | 0.0000E +00 | 0.0000E +00 | 0.0000E +00 |

Referring to FIG. 5, the first lens 1 has a negative optical power, with its object side surface 11 (the left side) being a planar surface, and image side surface 12 (the right side) being a concave surface. The second lens 2 has a positive optical power, with its object side surface 21 being a concave surface, and image side surface 22 being a convex surface. The third lens 3 has a positive optical power, with its third lens 3 being a biconvex lens, meaning both its object side surface 31 and image side surface 32 being convex surfaces. The fourth lens 4 has a negative optical power, with its object side surface 41 being a concave surface, and image side surface 42 being a W-shaped surface.

The imaging quality in this embodiment is excellent, with MTF data provided in FIG. 6. Table 5 provides the following parameters for each element in Embodiment 3: radius of curvature, thickness, refractive index, Abbe number, effective optical diameter, focal length f of the objective lens, field of view (FOV), F-number, and total track length (TTL). Table 6 provides the aspheric data of all elements.

### Fourth Embodiment (Embodiment 4):

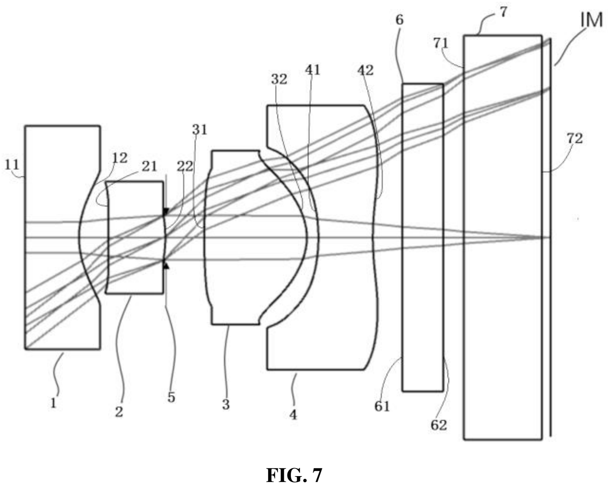

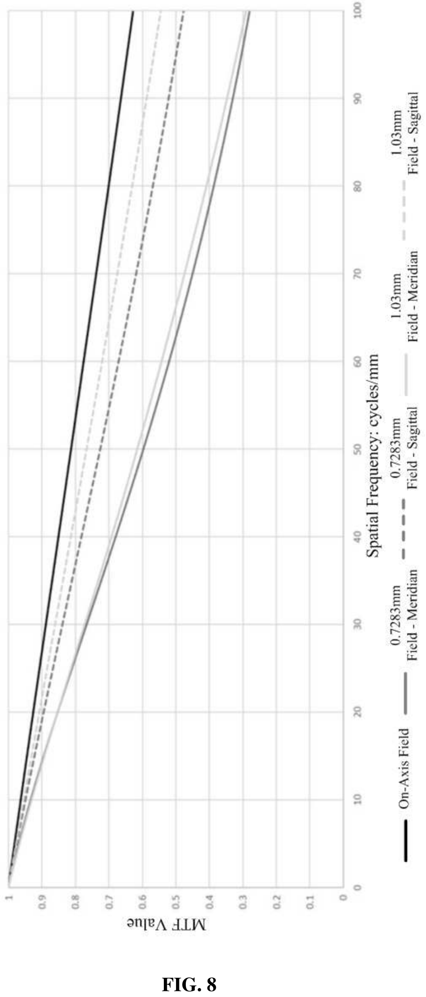

As shown in FIGS. 7 and 8, the left side is the object side, and the right side is the image side, with IM denoting the image plane. From the object side to the image plane, the optical components are arranged in the following order: the first lens 1, the second lens 2, the aperture stop 5, the third lens 3, the fourth lens 4, the optical filter 6, and the image sensor glass 7. In some embodiments, the optical filter 6 and the image sensor glass 7 are not necessary, and their use in the objective optical system may be selected based on requirements. Referring to FIG. 7, the first lens 1 has a negative optical power, with its object side surface 11 (the left side) being a planar surface, and image side surface 12 (the right side) being a concave surface. The second lens 2 has a positive optical power, with its object side surface 21 being a W-shaped surface, and image side surface 22 being a convex surface. The third lens 3 has a positive optical power and is a biconvex lens, with both its object side surface 31 and image side surface 32 being convex surfaces. The fourth lens 4 has a negative optical power, with its object side surface 41 being a concave surface, and the image side surface 42 being a W-shaped surface. The imaging quality in this embodiment is excellent, with MTF data provided in FIG. 8. Table 7 provides the following parameters for each element in Embodiment 4: radius of curvature, thickness, refractive index, Abbe number, effective optical diameter, focal length f of the objective lens, field of view (FOV), F-number, and total track length (TTL). Table 8 provides the aspheric data of all elements.

**Table 7 Construction Data of the Objective Optical System in Embodiment 4**

| f=0.754 | FOV=140° | F/4.903 | Image Height imgH=1.03 | | | | Total Track Length | |
|---|---|---|---|---|---|---|---|---|
| | | | TTL=2.697 | | | | | |
| Surface Number | Name | Surface Profile | Radius of Curvature | Thick ness | Refractiv e Index | Abbe Number | Effective Diameter | Focal Length |
| Object Plane | | | Infinite | 10.00 00 | | | 57.24 | |
| Object Side Surface 11 | First Lens 1 | Standard Spherica 1 Surface | Infinite | 0.276 7 | 1.5445 | 55.99 | 1.15 | -0.7881 |
| Image Side Surface 12 | | Even Aspheric Surface | 0.4291 | 0.150 1 | | | 0.59 | |
| Object Side Surface 21 | Second Lens 2 | Even Aspheric Surface | 3.2448 | 0.293 0 | 1.5445 | 55.99 | 0.48 | 1.0039 |
| Image Side Surface 22 | | Even Aspheric Surface | -0.6364 | -0.00 60 | | | 0.24 | |
| Aperture Plane | Aperture Stop 5 | Standard Spherica 1 Surface | Infinite | 0.205 7 | | | 0.23 | |
| Object Side Surface 31 | Third Lens 3 | Even Aspheric Surface | 3.2665 | 0.526 8 | 1.5445 | 55.99 | 0.61 | 0.5238 |
| Image Side Surface 32 | | Even Aspheric Surface | -0.2948 | 0.059 4 | | | 0.80 | |
| Object Side Surface 41 | Fourth Lens 4 | Even Aspheric Surface | -0.7697 | 0.279 2 | 1.651 | 21.51 | 0.83 | -0.6985 |
| Image Side Surface 42 | | Even Aspheric Surface | 1.2701 | 0.151 7 | | | 1.26 | |
| Object Side Surface 61 | Optical Filter 6 | Standard Spherica 1 Surface | Infinite | 0.210 0 | 1.5233 | 54.52 | 1.45 | |
| Image Side Surface 62 | | Standard Spherica 1 Surface | Infinite | 0.105 4 | | | 1.58 | |
| Object Side Surface 71 | Image Sensor Glass 7 | Standard Spherica 1 Surface | Infinite | 0.400 0 | 1.5168 | 64.2 | 1.70 | |
| Image Side Surface 72 | | Standard Spherica 1 Surface | Infinite | 0.045 0 | | | 1.98 | |
| IM | Image Plane | Standard Spherica 1 Surface | Infinite | | | | 2.05 | |

**Table 8 Aspheric Data of Aspheric Surfaces in Embodiment 4**

| Aspheric Coefficient | | | | | | | | |
|---|---|---|---|---|---|---|---|---|
| Surface Number | Conic Consta nt | A4 | A6 | A8 | A10 | A12 | A14 | A16 |
| Object Plane | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Object Side Surface 11 | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Image Side Surface 12 | -2.23E +00 | 2.5279E-01 | 1.0397E+ 00 | -1.3536E +02 | -4.6948E +02 | 1.0816E+ 04 | 1.0828E+ 05 | -1.1267E +06 |
| Object Side Surface 21 | -8.76E +01 | -4.7339E +00 | 4.4723E+ 01 | -1.2220E +03 | 1.5337E+ 04 | -4.6074E +04 | -6.0330E +05 | 3.9227E+ 06 |
| Image Side Surface 22 | 8.47E-0 1 | 6.8528E+ 00 | -6.4437E +02 | 9.7588E+ 03 | 1.3875E+ 06 | -1.6343E +07 | -2.8950E +09 | 5.5516E+ 10 |
| Aperture | 0.00E+ | 0.0000E+ | 0.0000E+ | 0.0000E+ | 0.0000E+ | 0.0000E+ | 0.0000E+ | 0.0000E+ |
| Plane | 00 | 00 | 00 | 00 | 00 | 00 | 00 | 00 |
| Object Side Surface 31 | -9.85E +01 | -4.4885E -01 | 3.0440E+ 01 | -6.7847E +02 | 8.8918E+ 03 | -3.6978E +04 | -1.3821E +05 | 1. 1976E+ 06 |
| Image Side Surface 32 | -1.19E +00 | 3.55E+00 | -5.73E+0 1 | 3.63E+02 | -1.03E+0 3 | 2.78E+03 | -1.23E+0 4 | 4.33E+04 |
| Object Side Surface 41 | 1.47E+ 00 | 2.63E+00 | -5.48E+0 1 | 1.55E+02 | 1.53E+03 | -1.20E+0 4 | 3.58E+04 | -5.29E+0 4 |
| Image Side Surface 42 | -4.70E +01 | 6.6450E-01 | -1.8560E +01 | 1.1230E+ 02 | -3.4965E +02 | 5.9694E+ 02 | -5.2785E +02 | 1.8156E+ 02 |
| Object Side Surface 61 | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Image Side Surface 62 | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Object Side Surface 71 | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Image Side Surface 72 | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Image Plane | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |

### Fifth Embodiment (Embodiment5):

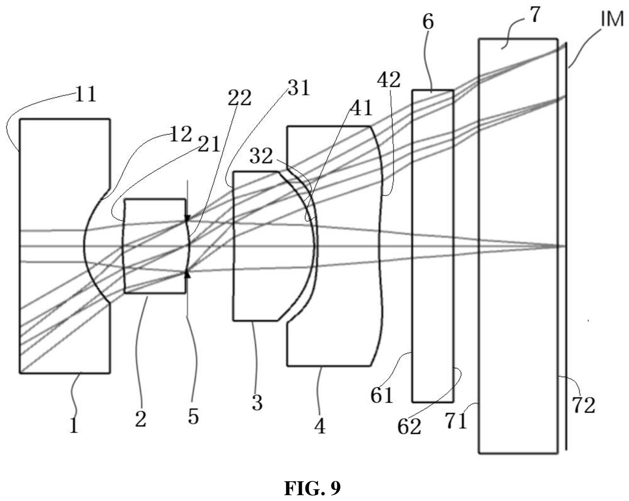

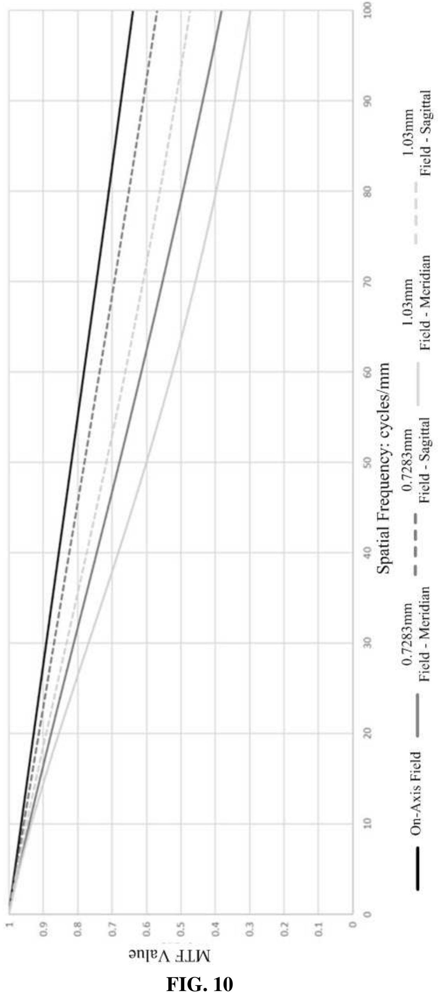

As shown in FIGS. 9 and 10, the left side is the object side and the right side is the image side, with IM denoting the image plane. From the object side to the image plane, optical components are arranged in the following order: the first lens 1, the second lens 2, the aperture stop 5, the third lens 3, the fourth lens 4, the optical filter 6 and the image sensor glass 7. In some embodiments, the optical filter 6 and the image sensor glass 7 are not necessary, and their use in the objective optical system may be selected based on requirements.

**Table 9 Construction Data of the Objective Optical System in Embodiment 5**

| f=0.753 | FOV=140° | F/4.827 | Image Height imgH=1.03 | | | Total Track Length | | |
|---|---|---|---|---|---|---|---|---|
| | | | TTL=2.782 | | | | | |
| Surface Number | Name | Surface Profile | Radius of Curvature | Thickness | Refractive Index | Abbe Number | Effective Diameter | Focal Length |
| Object Plane | | | Infinite | 10.0000 | | | 57.49 | |
| Object Side Surface 11 | First Lens 1 | Standard Spherical Surface | Infinite | 0.3271 | 1.5445 | 55.99 | 1.29 | -0.6057 |
| Image Side Surface 12 | | Even Aspheric Surface | 0.3298 | 0.1969 | | | 0.58 | |
| Object Side Surface 21 | Second Lens 2 | Even Aspheric Surface | 1.1039 | 0.3387 | 1.5445 | 55.99 | 0.48 | 0.68 |
| Image Side Surface 22 | | Even Aspheric Surface | -0.4969 | -0.0138 | | | 0.27 | |
| Apertur e Plane | Aperture Stop 5 | Standard Spherical Surface | 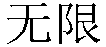 | 0.2414 | | | 0.26 | |
| Object Side Surface 31 | Third Lens 3 | Even Aspheric Surface | -13.3880 | 0.4082 | 1.5445 | 55.99 | 0.57 | 0.8685 |
| Image Side Surface 32 | | Even Aspheric Surface | -0.4617 | 0.0169 | | | 0.76 | |
| Object Side Surface 41 | Fourth Lens 4 | Even Aspheric Surface | -1.5708 | 0.3138 | 1.651 | 21.51 | 0.78 | -1.3758 |
| Image Side Surface 42 | | Even Aspheric Surface | 2.2475 | 0.1686 | | | 1.22 | |
| Object Side Surface 61 | Optical Filter 6 | Standard Spherical Surface | Infinite | 0.2100 | 1.5233 | 54.52 | 1.45 | |
| Image Side Surface 62 | | Standard Spherical Surface | Infinite | 0.1295 | | | 1.59 | |
| Object Side Surface 71 | Image Sensor Glass 7 | Standard Spherical Surface | Infinite | 0.4000 | 1.5168 | 64.2 | 1.73 | |
| Image Side Surface 72 | | Standard Spherical Surface | Infinite | 0.0450 | | | 2.01 | |
| IM | Image Plane | Standard Spherical Surface | Infinite | | | | 2.08 | |

Referring to FIG. 9, the first lens 1 has a negative optical power, with its object side surface 11 (the left side) being a planar surface, and image side surface 12 (the right side) being a concave surface. The second lens 2 has a positive optical power, with its object side surface 21 being a W-shaped surface, and image side surface 22 being a convex surface. The third lens 3 has a positive optical power, with its object side surface 31 being a concave surface, and image side surface 32 being a convex surface. The fourth lens 4 has a negative optical power, with its object side surface 41 being a concave surface, and image side surface 42 being a W-shaped surface.

The imaging quality in this embodiment is excellent, with MTF data provided in FIG. 10. Table 9 provides the following parameters for each element in Embodiment 5: radius of curvature, thickness, refractive index, Abbe number, effective optical diameter, focal length f of the objective lens, field of view (FOV), F-number, and total track length (TTL). Table 10 provides the aspheric data of all elements.

**Table 10 Aspheric Data of Aspheric Surfaces in Embodiment 5**

| Aspheric Coefficient | | | | | | | | |
|---|---|---|---|---|---|---|---|---|
| Surfac e Numb er | Conic Constant | A4 | A6 | A8 | A10 | A12 | A14 | A16 |
| Object Plane | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Object Side Surfac e 11 | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Image Side Surfac e 12 | -1.51E+ 00 | 1.7834E+ 00 | 2.0215E+ 01 | -2.8158E +02 | -1.2491E +03 | 1.8845E+ 04 | 2.4992E+ 05 | -2.4626E +06 |
| Object Side Surfac e 21 | -1.49E+ 01 | -2.5495E +00 | -3.9080E +00 | -1.1818E +03 | 1.9854E+ 04 | -8.8874E +04 | -1.6769E +06 | 1.7589E+ 07 |
| Image Side Surfac e 22 | 2.27E+ 00 | 5.9689E+ 00 | -4.3397E +02 | 5.9543E+ 03 | 1.1330E+ 06 | -1.7299E +07 | -2.4756E +09 | 6.7675E+ 10 |
| Apertu re Plane | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Object Side Surface 31 | -1.00E+ 02 | -3.2708E-01 | 2.8978E+ 01 | -8.1721E +02 | 8.5863E+ 03 | -2.7708E +04 | -2.6359E +03 | -3.9287E +05 |
| Image Side Surfac e 32 | -4.60E-01 | 2.30E+00 | -5.95E+0 1 | 3.61E+02 | -1.06E+0 3 | 2.97E+03 | -6.43E+0 3 | 7.05E+04 |
| Object Side Surfac e 41 | 8.87E+ 00 | 6.55E-01 | -6.12E+0 1 | 1.22E+02 | 1.40E+03 | -1.21E+0 4 | 3.91E+04 | 1.81E+04 |
| Image Side Surfac e 42 | -1.89E+ 01 | 5.3926E-01 | -1.9013E +01 | 1.1186E+ 02 | -3.4941E +02 | 5.9986E+ 02 | -5.2064E +02 | 1.7538E+ 02 |
| Object Side Surfac e 61 | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Image Side Surfac e 62 | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Object Side Surfac e 71 | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Image Side Surfac e 72 | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Image Plane | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |

### Sixth Embodiment (Embodiment 6)

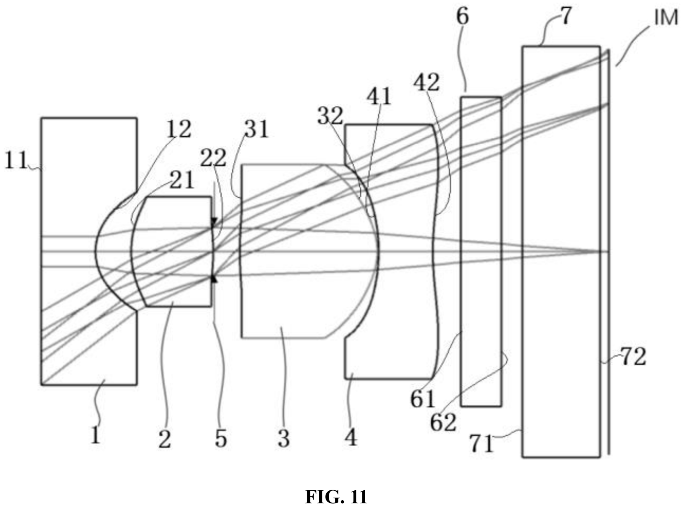

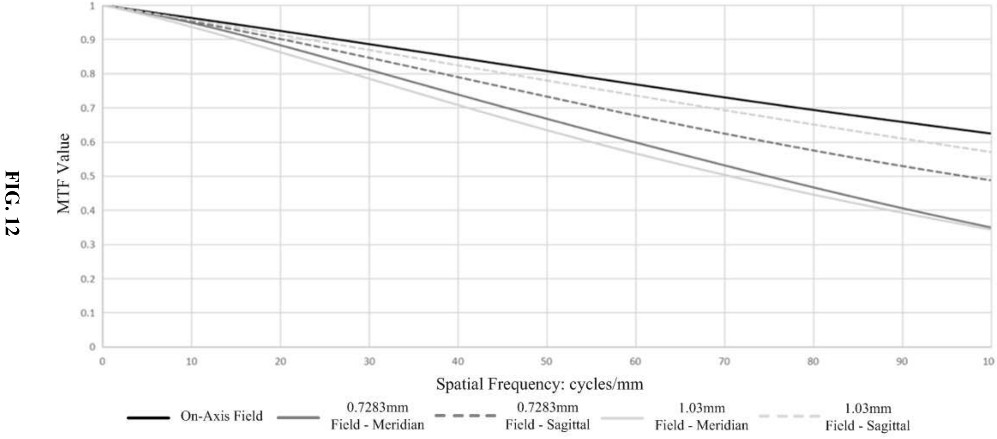

As shown in FIGS. 11 and 12, the left side is the object side and the right side is the image side, with IM denoting the image plane.

**Table 11 Construction Data of the Objective Optical System in Embodiment 6**

| f=0.719 | FOV=140° | F/4.869 | Image Height imgH=1.03 | | | Total Track Length | | |
|---|---|---|---|---|---|---|---|---|
| | | | TTL=2.918 | | | | | |
| Surface Number | Name | Surface Profile | Radius of Curvatur e | Thicknes s | Refractiv e Index | Abbe Number | Effective Diameter | Focal Length |
| Object Plane | | | Infinite | 10.0000 | | | 57.36 | |
| Object Side Surface 11 | First Lens 1 | Standard Spherical Surface | Infinite | 0.2798 | 1.5445 | 55.99 | 1.37 | -0.4623 |
| Image Side Surface 12 | | Even Aspheric Surface | 0.2517 | 0.1834 | | | 0.61 | |
| Object Side Surface 21 | Second Lens 2 | Even Aspheric Surface | 0.4522 | 0.4217 | 1.5445 | 55.99 | 0.56 | 0.6443 |
| Image Side Surface 22 | | Even Aspheric Surface | -1.0504 | -0.0020 | | | 0.26 | |
| Apertur e Plane | Aperture Stop 5 | Standard Spherical Surface | Infinite | 0.1384 | | | 0.25 | |
| Object Side Surface 31 | Third Lens 3 | Even Aspheric Surface | 1.4831 | 0.7069 | 1.5445 | 55.99 | 0.50 | 0.5611 |
| Image Side Surface 32 | | Even Aspheric Surface | -0.3201 | 0.0076 | | | 0.89 | |
| Object Side Surface 41 | Fourth Lens 4 | Even Aspheric Surface | -0.7406 | 0.2777 | 1.651 | 21.51 | 0.89 | -0.6925 |
| Image Side Surface 42 | | Even Aspheric Surface | 1.3221 | 0.1429 | | | 1.31 | |
| Object Side Surface 61 | Optical Filter 6 | Standard Spherical Surface | Infinite | 0.2100 | 1.5233 | 54.52 | 1.46 | |
| Image Side Surface 62 | | Standard Spherical Surface | Infinite | 0.1062 | | | 1.59 | |
| Object Side Surface 71 | Image Sensor Glass 7 | Standard Spherical Surface | Infinite | 0.4000 | 1.5168 | 64.2 | 1.70 | |
| Image Side Surface 72 | | Standard Spherical Surface | Infinite | 0.0450 | | | 2.01 | |
| IM | Image Plane | Standard Spherical Surface | Infinite | | | | 2.08 | |

From the object side to the image plane, the optical components are arranged in the following order: the first lens 1, the second lens 2, the aperture stop 5, the third lens 3, the fourth lens 4, the optical filter 6, and the image sensor glass 7. In some embodiments, the optical filter 6 and the image sensor glass 7 are not necessary, and their use in the objective optical system may be selected based on requirements.

**Table 12 Aspheric Data of Aspheric Surfaces in Embodiment 6**

| Aspheric Coefficient | | | | | | | | |
|---|---|---|---|---|---|---|---|---|
| Surfac e Numb er | Conic Constan t | A4 | A6 | A8 | A10 | A12 | A14 | A16 |
| Object Plane | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Object Side Surfac e 11 | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Image Side Surfac e 12 | -1.32E+ 00 | 2.9129E+ 00 | 5.4427E+ 01 | -2.9166E +02 | -2.2869E +03 | 1.8112E+ 04 | 4.3133E+ 05 | -4.3378E +06 |
| Object Side Surfac e 21 | -4.47E+ 00 | 2.3970E+ 00 | 5.9445E+ 01 | -1.4835E +03 | 1.0977E+ 04 | -7.2851E +04 | 6.2685E+ 05 | -3.5458E +06 |
| Image Side Surfac e 22 | -1.55E+ 01 | 7.4456E+ 00 | -8.4259E +02 | 5.3746E+ 03 | 1.5374E+ 06 | 6.2613E+ 05 | -2.5401E +09 | 1.2516E+ 10 |
| Apertu re Plane | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Object Side Surfac e 31 | -1.00E+ 02 | -5.4891E -02 | 8.3732E-01 | -8.8570E +02 | 1.8343E+ 04 | -9.4045E +04 | -3.2003E +06 | 3.8661E+ 07 |
| Image Side Surfac e 32 | -2.29E+ 00 | 2.85E+00 | -5.52E+0 1 | 3.34E+02 | -1.16E+0 3 | 3.09E+03 | -1.10E+0 4 | 1.99E+04 |
| Object Side Surfac e 41 | 7.85E-0 1 | 4.16E+00 | -4.97E+0 1 | 1.10E+02 | 1.73E+03 | -1.39E+0 4 | 3.51E+04 | -2.65E+0 4 |
| Image Side Surfac e 42 | -7.05E+ 01 | 9.0692E-01 | -1.8289E +01 | 1.1707E+ 02 | -3.8724E +02 | 6.7117E+ 02 | -5.5727E +02 | 1.5542E+ 02 |
| Object Side Surfac e 61 | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Image Side Surfac e 62 | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Object Side Surfac e 71 | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Image Side Surfac e 72 | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Image Plane | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |

Referring to FIG. 11, the first lens 1 has a negative optical power, with its object side surface 11 (the left side) being a planar surface, and image side surface 12 (the right side) being a concave surface. The second lens 2 has a positive optical power and is a biconvex lens, with both its object side surface 21 and image side surface 22 being convex surfaces. The third lens 3 has a positive optical power and is a biconvex lens, with both its object side surface 31 and image side surface 32 being convex surfaces. The fourth lens 4 has a negative optical power, with its object side surface 41 being a concave surface, and image side surface 42 is a W-shaped surface. The imaging quality in this embodiment is excellent, with MTF data provided in FIG. 12. Table 11 provides the following parameters for each element in Embodiment 6: radius of curvature, thickness, refractive index, Abbe number, effective optical diameter, focal length f of the objective lens, field of view (FOV), F-number, and total track length (TTL). Table 12 provides the aspheric data of all elements.

### Seventh Embodiment (Embodiment 7):

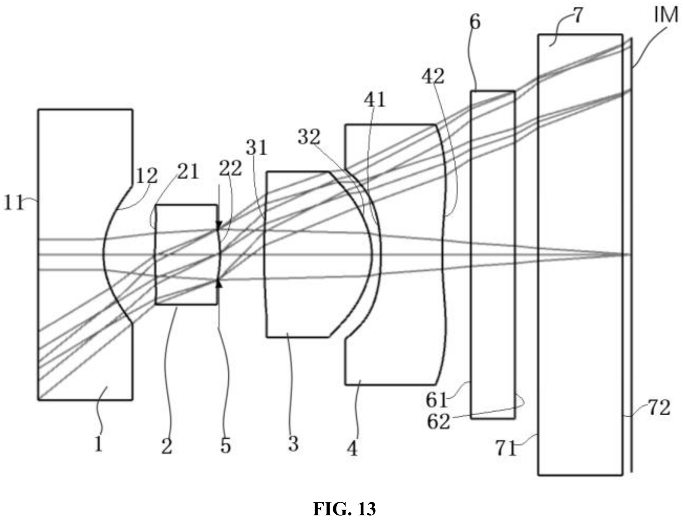

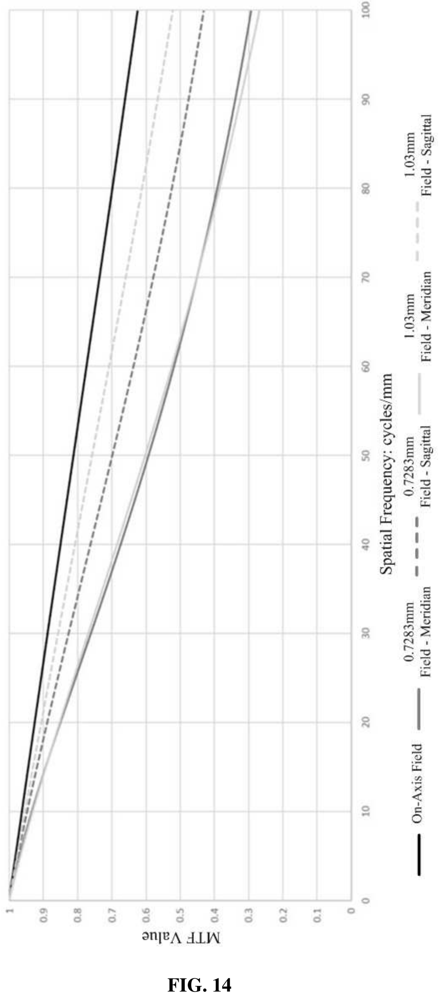

As shown in FIGS. 13 and 14, the left side is the object side, and the right side is the image side, with IM denoting the image plane. From the object side to the image plane, the optical components are arranged in the following order: the first lens 1, the second lens 2, the aperture stop 5, the third lens 3, the fourth lens 4, the optical filter 6, and the image sensor glass 7. In some embodiments, the optical filter 6 and the image sensor glass 7 are not necessary, and their use in the objective optical system may be selected based on requirements. Referring to FIG. 13, the first lens 1 has a negative optical power, with its object side surface 11 (the left side) being a planar surface, and image side surface 12 (the right side) being a concave surface. The second lens 2 has a positive optical power and is a biconvex lens, with both its object side surface 21 and image side surface 22 being convex surfaces. The third lens 3 has a positive optical power and is a biconvex lens, with both its object side 31 and image side surface 32 being convex surfaces. The fourth lens 4 has a negative optical power and is a concave surface, with its image side surface 42 being a W-shaped surface. The imaging quality in this embodiment is excellent, with MTF data provided in FIG. 14. Table 13 provides the following parameters for each element in Embodiment 7: radius of curvature, thickness, refractive index, Abbe number, effective optical diameter, focal length f of the objective lens, field of view (FOV), F-number, and total track length (TTL). Table 14 provides the aspheric data of all elements.

**Table 13 Construction Data of the Objective Optical System in Embodiment 7**

| f=0.709 | FOV=150° | F/4.999 | | Image Height imgH=1.03 | | Total Track Length | | |
|---|---|---|---|---|---|---|---|---|
| | | | TTL=2.822 | | | | | |
| Surface Number | Name | Surface Profile | Radius of Curvatur e | Thicknes s | Refractiv e Index | Abbe Numbe r | Effective Diameter | Focal Length |
| Object Plane | | | Infinite | 10.0000 | | | 78.14 | |
| Object Side Surface 11 | First Lens 1 | Standard Spherical Surface | Infinite | 0.3111 | 1.5445 | 55.99 | 1.38 | -0.6657 |
| Image Side Surface 12 | | Even Aspheric Surface | 0.3625 | 0.2444 | | | 0.66 | |
| Object Side Surface 21 | Second Lens 2 | Even Aspheric Surface | 1.6056 | 0.3094 | 1.5445 | 55.99 | 0.47 | 0.8319 |
| Image Side Surface 22 | | Even Aspheric Surface | -0.5881 | -0.0101 | | | 0.24 | |
| Apertur e Plane | Aperture Stop 5 | Standard Spherical Surface | Infinite | 0.2198 | | | 0.24 | |
| Object Side Surface 31 | Third Lens 3 | Even Aspheric Surface | 3.3422 | 0.5119 | 1.5445 | 55.99 | 0.58 | 0.6147 |
| Image Side Surface 32 | | Even Aspheric Surface | -0.3519 | 0.0435 | | | 0.79 | |
| Object Side Surface 41 | Fourth Lens 4 | Even Aspheric Surface | -0.8404 | 0.2908 | 1.651 | 21.51 | 0.81 | -0.8321 |
| Image Side Surface 42 | | Even Aspheric Surface | 1.7313 | 0.1367 | | | 1.24 | |
| Object Side Surface 61 | Optical Filter 6 | Standard Spherical Surface | Infinite | 0.2100 | 1.5233 | 54.52 | 1.42 | |
| Image Side Surface 62 | | Standard Spherical Surface | Infinite | 0.1092 | | | 1.56 | |
| Object Side Surface 71 | Image Sensor Glass 7 | Standard Spherical Surface | Infinite | 0.4000 | 1.5168 | 64.2 | 1.68 | |
| Image Side Surface 72 | | Standard Spherical Surface | Infinite | 0.0450 | | | 1.99 | |
| IM | Image Plane | Standard Spherical Surface | Infinite | | | | 2.06 | |

**Aspheric surface data of the seventh embodiment in Table 14**

| Aspheric Coefficient | | | | | | | | |
|---|---|---|---|---|---|---|---|---|
| Surfac e Numb er | Conic Constan t | A4 | A6 | A8 | A10 | A12 | A14 | A16 |
| Object Plane | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Object Side Surfac e 11 | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Image Side Surfac e 12 | -1.71E+ 00 | 9.5391E-01 | 7.6258E+ 00 | -1.2483E +02 | -6.1983E +02 | 7.2032E+ 03 | 7.9733E+ 04 | -6.8700E +05 |
| Object Side Surfac e 21 | -1.00E+ 02 | -2.5532E +00 | 2.5960E+ 01 | -1.5504E +03 | 1.2240E+ 04 | 2.7511E+ 04 | 1.6341E+ 06 | -2.8788E +07 |
| Image Side Surfac e 22 | 2.70E+ 00 | 4.0028E+ 00 | -2.6870E +02 | -2.3840E +03 | 9.8055E+ 05 | -5.7616E +06 | -2.0312E +09 | 4.3580E+ 10 |
| Apertu re Plane | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Object Side Surfac e 31 | 2.96E+ 00 | -3.9307E-01 | 2.1378E+ 01 | -7.3962E +02 | 8.8366E+ 03 | -3.2819E +04 | -1.0004E +05 | 1.2462E+ 06 |
| Image Side Surfac e 32 | -1.13E+ 00 | 3.42E+00 | -5.64E+0 1 | 3.54E+02 | -1.05E+0 3 | 2.89E+03 | -1.18E+0 4 | 3.36E+04 |
| Object Side Surfac e 41 | 1.90E+ 00 | 1.84E+00 | -5.66E+0 1 | 1.61E+02 | 1.54E+03 | -1.20E+0 4 | 3.54E+04 | -5.23E+0 4 |
| Image Side Surfac e 42 | -7.64E+ 01 | 6.5450E-01 | -1.8588E +01 | 1.1233E+ 02 | -3.4972E +02 | 5.9636E+ 02 | -5.2862E +02 | 1.8586E+ 02 |
| Object Side Surfac e 61 | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Image Side Surfac e 62 | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Object Side Surfac e 71 | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Image Side Surfac e 72 | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Image Plane | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |

### Eighth Embodiment (Embodiment 8):

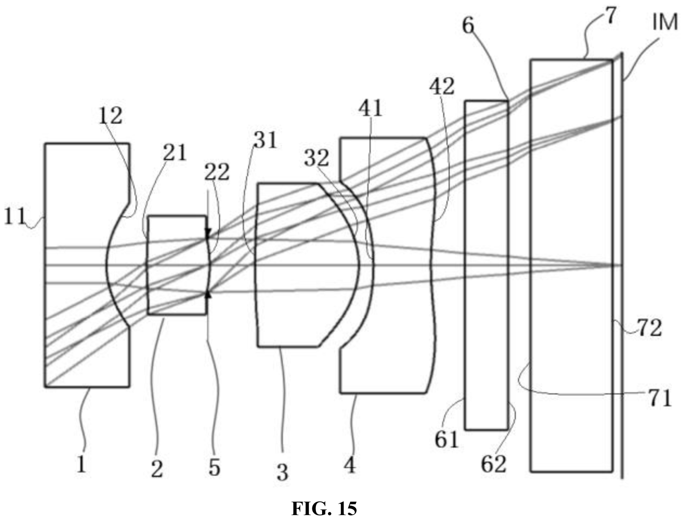

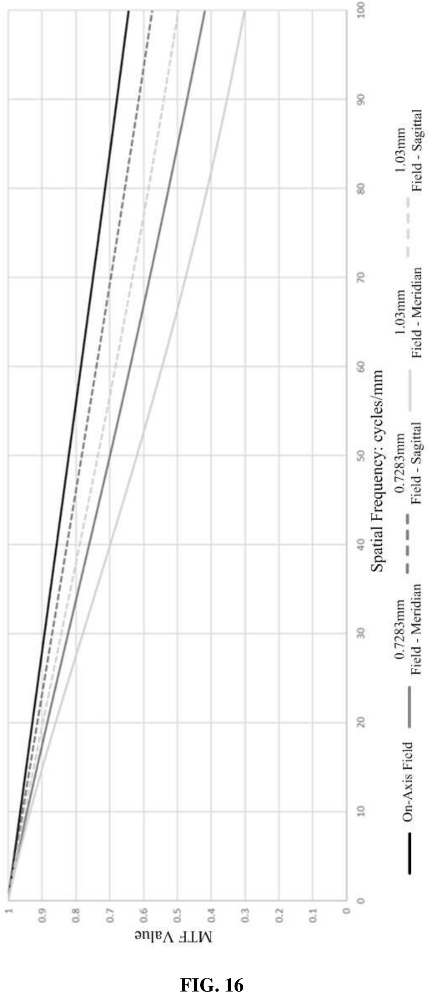

As shown in FIGS. 15 and 16, the left side is the object side and the right side is the image side, with IM denoting the image plane. From the object side to the image plane, the optical components are arranged in the following order: the first lens 1, the second lens 2, the aperture stop 5, the third lens 3, the fourth lens 4, the optical filter 6, and the image sensor glass 7. In some embodiments, the filter 6 and the image sensor glass 7 are not necessary, and their use in the objective optical system may be selected based on requirements. Referring to FIG. 15, the first lens 1 has a negative optical power, with its object side surface 11 (the left side) being a planar surface and its image side surface 12 (the right side) being a concave surface. The second lens 2 has a positive optical power and is a biconvex lens, with both its object side surface 21 and image side surface 22 being convex surfaces. The third lens 3 has a positive optical power and is also a biconvex lens, with both its object side surface 31 and image side surface 32 being convex surfaces. The fourth lens 4 has a negative optical power, with its object side 41 being a concave surface and its image side surface 42 being a W-shaped surface. The imaging quality in this embodiment is excellent, with MTF data provided in FIG. 16.

**Table 15 Construction Data of the Objective Optical System in Embodiment 8**

| f=0.793 | FOV=125° | F/4.792 | Image Height imgH=1.03 | | | Total Track Length | | |
|---|---|---|---|---|---|---|---|---|
| | | | TTL=2.815 | | | | | |
| Surface Number | Name | Surface Profile | Radius of Curvatu re | Thickne ss | Refracti ve Index | Abbe Numb er | Effectiv e Diamete r | Focal Length |
| Object Plane | | | Infinite | 10.0000 | | | 40.16 | |
| Object Side Surface 11 | First Lens 1 | Standard Spherical Surface | Infinite | 0.3015 | 1.5445 | 55.99 | 1.19 | -0.734 2 |
| Image Side Surface 12 | | Even Aspheric Surface | 0.3998 | 0.1970 | | | 0.61 | |
| Object Side Surface 21 | Second Lens 2 | Even Aspheric Surface | 1.6194 | 0.3062 | 1.5445 | 55.99 | 0.48 | 0.8534 |
| Image Side Surface 22 | | Even Aspheric Surface | -0.6083 | -0.0094 | | | 0.27 | |
| Aperture Plane | Aperture Stop 5 | Standard Spherical Surface | Infinite | 0.2314 | | | 0.26 | |
| Object Side Surface 31 | Third Lens 3 | Even Aspheric Surface | 4.3668 | 0.5057 | 1.5445 | 55.99 | 0.59 | 0.6525 |
| Image Side Surface 32 | | Even Aspheric Surface | -0.3710 | 0.0712 | | | 0.80 | |
| Object Side Surface 41 | Fourth Lens 4 | Even Aspheric Surface | -0.9249 | 0.2802 | 1.651 | 21.51 | 0.82 | -0.870 4 |
| Image Side Surface 42 | | Even Aspheric Surface | 1.6372 | 0.1665 | | | 1.25 | |
| Object Side Surface 61 | Optical Filter 6 | Standard Spherical Surface | Infinite | 0.2100 | 1.5233 | 54.52 | 1.46 | |
| Image Side Surface 62 | | Standard Spherical Surface | Infinite | 0.1098 | | | 1.61 | |
| Object Side Surface 71 | Image Sensor Glass 7 | Standard Spherical Surface | Infinite | 0.4000 | 1.5168 | 64.2 | 1.73 | |
| Image Side Surface 72 | | Standard Spherical Surface | Infinite | 0.0450 | | | 2.01 | |
| IM | Image Plane | Standard Spherical Surface | Infinite | | | | 2.08 | |

**Table 16 Aspheric Data of Aspheric Surfaces in Embodiment 8**

| Aspheric Coefficient | | | | | | | | |
|---|---|---|---|---|---|---|---|---|
| Surfac e Numb er | Conic Constan t | A4 | A6 | A8 | A10 | A12 | A14 | A16 |
| Object Plane | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Object Side Surfac e 11 | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Image Side Surfac e 12 | -1.73E+ 00 | 8.3654E-01 | 5.9730E+ 00 | -1.0669E +02 | -4.4546E +02 | 8.6292E+ 03 | 8.1001E+ 04 | -8.4507E +05 |
| Object Side Surfac e 21 | -9.98E+ 01 | -2.0964E +00 | 3.2109E+ 01 | -1.5198E +03 | 1.2736E+ 04 | 1.8360E+ 04 | 1.4823E+ 06 | -2.5201E +07 |
| Image Side Surfac e 22 | 2.12E+ 00 | 5.3095E+ 00 | -4.0010E +02 | -2.0962E +03 | 1. 1395E+ 06 | -3.3687E +05 | -1.9530E +09 | 2.8944E+ 10 |
| Apertu re Plane | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Object Side Surfac e 31 | 1.00E+ 02 | -5.3034E-01 | 2.6000E+ 01 | -7.0835E +02 | 8.8334E+ 03 | -3.5319E +04 | -1.3373E +05 | 9.4332E+ 05 |
| Image Side Surfac e 32 | -1.07E+ 00 | 3.31E+00 | -5.50E+0 1 | 3.70E+02 | -1.04E+0 3 | 2.59E+03 | -1.37E+0 4 | 2.84E+04 |
| Object Side Surfac e 41 | 1.40E+ 00 | 2.01E+00 | -5.81E+0 1 | 1.57E+02 | 1.57E+03 | -1.19E+0 4 | 3.48E+04 | -6.59E+0 4 |
| Image Side Surfac e 42 | -4.65E+ 01 | 6.9635E-01 | -1.9077E +01 | 1. 1264E+ 02 | -3.4487E +02 | 5.8736E+ 02 | -5.3022E +02 | 1.9358E+ 02 |
| Object Side Surfac e 61 | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Image Side Surfac e 62 | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Object Side Surfac e 71 | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Image Side Surfac e 72 | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |
| Image Plane | 0.00E+ 00 | 0.0000E+ 00 | 0.0000E+ 00 | 0.0000E+ 00 | 0.0000E+ 00 | 0.0000E+ 00 | 0.0000E+ 00 | 0.0000E+ 00 |

Table 15 provides the following parameters for each element in Embodiment 8: curvature radius, thickness, refractive index, Abbe number, effective optical diameter, focal length f of the objective lens, field of view (FOV), F-number, and total track length (TTL). Table 16 provides the aspheric data of all elements.

### Ninth Embodiment (Embodiment 9):

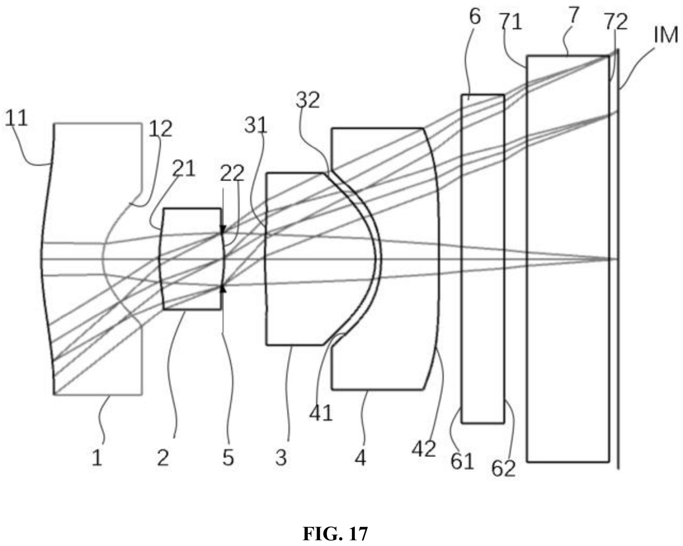

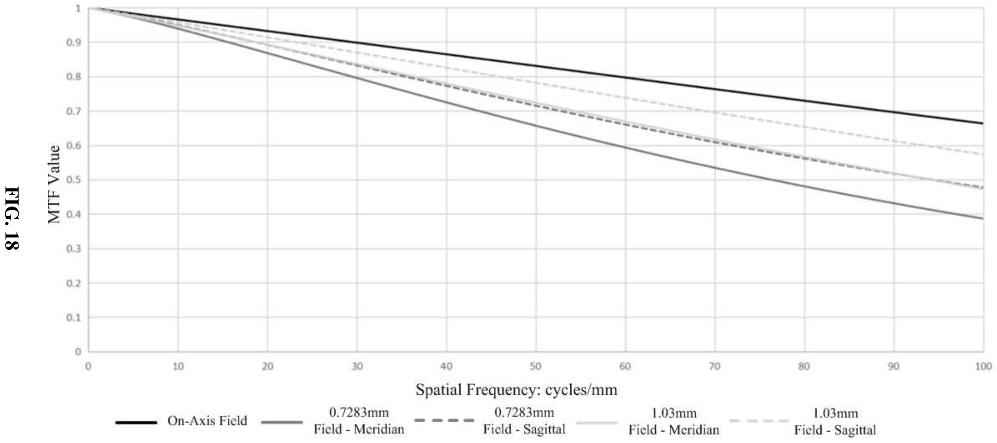

As shown in FIGS. 17 and 18, the left side is the object side and the right side is the image side, with IM denoting the image plane. From the object side to the image plane, the optical components are arranged in the following order: the first lens 1, the second lens 2, the aperture stop 5, the third lens 3, the fourth lens 4, the optical filter 6, and the image sensor glass 7. In some embodiments, the optical filter 6 and the image sensor glass 7 are not necessary, and their use in the objective optical system may be selected based on requirements..

**Table 17 Construction Data of the Objective Optical System in Embodiment 9**

| f=0.747 | FOV=140° | F/4.556 | Image Height imgH=1.03 | | | Total Track Length | | |
|---|---|---|---|---|---|---|---|---|
| | | | TTL=2.813 | | | | | |
| Surfac e Numbe r | Name | Surface Profile | Radius of Curvatu re | Thickne ss | Refracti ve Index | Abbe Numb er | Effectiv e Diamet er | Focal Length |
| Object Plane | | | Infinite | 10.0000 | | | 58.00 | |
| Object Side Surfac e 11 | First Lens 1 | Even Aspheric Surface | 1.2029 | 0.2991 | 1.5445 | 55.99 | 1.33 | -0.624 1 |
| Image Side Surfac e 12 | | Even Aspheric Surface | 0.2417 | 0.2759 | | | 0.66 | |
| Object Side Surfac e 21 | Second Lens 2 | Even Aspheric Surface | 0.8101 | 0.3157 | 1.5445 | 55.99 | 0.49 | 0.7435 |
| Image Side Surfac e 22 | | Even Aspheric Surface | -0.6982 | -0.0082 | | | 0.27 | |
| Apertu re Plane | Aperture Stop 5 | Standard Spherical Surface | 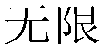 | 0.2074 | | | 0.26 | |
| Object Side Surfac e 31 | Third Lens 3 | Even Aspheric Surface | 2.0622 | 0.5383 | 1.5445 | 55.99 | 0.57 | 0.5946 |
| Image Side Surfac e 32 | | Even Aspheric Surface | -0.3487 | 0.0281 | | | 0.84 | |
| Object Side Surfac e 41 | Fourth Lens 4 | Even Aspheric Surface | -0.5421 | 0.2816 | 1.651 | 21.51 | 0.87 | -0.840 7 |
| Image Side Surfac e 42 | | Even Aspheric Surface | -69.367 2 | 0.1099 | | | 1.28 | |
| Object Side Surfac e 61 | Optical Filter 6 | Standard Spherical Surface | Infinite | 0.2100 | 1.5233 | 54.52 | 1.47 | |
| Image Side Surfac e 62 | | Standard Spherical Surface | Infinite | 0.1099 | | | 1.61 | |
| Object Side Surfac e 71 | Image Sensor Glass 7 | Standard Spherical Surface | Infinite | 0.4000 | 1.5168 | 64.2 | 1.72 | |
| Image Side Surfac e 72 | | Standard Spherical Surface | Infinite | 0.0450 | | | 1.99 | |
| IM | Image Plane | Standard Spherical Surface | Infinite | | | | 2.06 | |

**Table 18 Aspheric Data of Aspheric Surfaces in Embodiment 9**

| Aspheric Coefficient | | | | | | | | |
|---|---|---|---|---|---|---|---|---|
| Surfac e Numb er | Conic Constan t | A4 | A6 | A8 | A10 | A12 | A14 | A16 |
| Object Plane | 0.00E+0 0 | 0.0000E+ 00 | 0.0000E+ 00 | 0.0000E+ 00 | 0.0000E+ 00 | 0.0000E+ 00 | 0.0000E+ 00 | 0.0000E+ 00 |
| Object Side Surfac e 11 | -2.36E+ 01 | -1.3642E-01 | -1.5794E-01 | -8.8184E-02 | 4.4673E-0 1 | 1.8340E-0 1 | 0.0000E+ 00 | 0.0000E+ 00 |
| Image Side Surfac e 12 | -1.79E+ 00 | 3.6998E+ 00 | -5.4136E +00 | -7.5512E +01 | -9.6268E +02 | 3.2461E+ 03 | 0.0000E+ 00 | 0.0000E+ 00 |
| Object Side Surfac e 21 | -2.03E+ 01 | 8.8953E-01 | -4.3612E +01 | -3.6597E +02 | 5.3087E+ 03 | -4.1425E +04 | 0.0000E+ 00 | 0.0000E+ 00 |
| Image Side Surfac e 22 | 1.23E+0 1 | 4.8940E+ 00 | -4.5736E +02 | 3.6641E+ 04 | -8.5334E +05 | 2.3961E+ 06 | 0.0000E+ 00 | 0.0000E+ 00 |
| Apertu re Plane | 0.00E+0 0 | 0.0000E+ 00 | 0.0000E+ 00 | 0.0000E+ 00 | 0.0000E+ 00 | 0.0000E+ 00 | 0.0000E+ 00 | 0.0000E+ 00 |
| Object Side Surfac e 31 | -5.67E+ 01 | -8.7051E-01 | -3.0739E +00 | -4.7862E +01 | 1.2912E+ 03 | -1.9506E +04 | 0.0000E+ 00 | 0.0000E+ 00 |
| Image Side Surfac e 32 | -6.83E-01 | 1.00E-01 | 3.46E+00 | 1.04E+01 | 9.18E+01 | -3.39E+0 2 | 0.00E+00 | 0.00E+00 |
| Object Side Surfac e 41 | 1.93E-0 1 | -1.87E+0 0 | -2.82E+0 0 | 2.83E+01 | 1.21E+02 | 2.83E+02 | 0.00E+00 | 0.00E+00 |
| Image Side Surfac e 42 | 1.00E+0 2 | -4.1500E-01 | -2.8662E-01 | 1.1595E-0 1 | 1.0070E+ 00 | 2.5906E-0 1 | 0.0000E+ 00 | 0.0000E+ 00 |
| Object Side Surfac e 61 | 0.00E+0 0 | 0.0000E+ 00 | 0.0000E+ 00 | 0.0000E+ 00 | 0.0000E+ 00 | 0.0000E+ 00 | 0.0000E+ 00 | 0.0000E+ 00 |
| Image Side Surfac e 62 | 0.00E+0 0 | 0.0000E+ 00 | 0.0000E+ 00 | 0.0000E+ 00 | 0.0000E+ 00 | 0.0000E+ 00 | 0.0000E+ 00 | 0.0000E+ 00 |
| Object Side Surfac e 71 | 0.00E+0 0 | 0.0000E+ 00 | 0.0000E+ 00 | 0.0000E+ 00 | 0.0000E+ 00 | 0.0000E+ 00 | 0.0000E+ 00 | 0.0000E+ 00 |
| Image Side Surfac e 72 | 0.00E+0 0 | 0.0000E+ 00 | 0.0000E+ 00 | 0.0000E+ 00 | 0.0000E+ 00 | 0.0000E+ 00 | 0.0000E+ 00 | 0.0000E+ 00 |
| Image Plane | 0.00E+0 0 | 0.0000E+ 00 | 0.0000E+ 00 | 0.0000E+ 00 | 0.0000E+ 00 | 0.0000E+ 00 | 0.0000E+ 00 | 0.0000E+ 00 |

Referring to FIG. 17, the first lens 1 has a negative optical power, with its object side surface 11 (the left side) being a convex surface and its image side surface 12 (the right side) being a concave surface. The second lens 2 has a positive optical power and is a biconvex lens, with both its object side surface 21 and image side surface 22 being convex surfaces. The third lens 3 has a positive optical power and is also a biconvex lens, with both its object side 31 and image side surface 32 being convex surfaces. The fourth lens 4 has a negative optical power, with its object side 41 being a concave surface and its image side surface 42 being a convex surface. The imaging quality in this embodiment is excellent, with MTF data provided in FIG. 18. Table 17 provides the following parameters for each element in Embodiment 9: curvature radius, thickness, refractive index, Abbe number, effective optical diameter, focal length f of the objective lens, field of view (FOV), F-number, and total track length (TTL). Table 18 provides the aspheric data of all elements.

The endoscopic objective optical systems described in Embodiments 1 to 9 meet the data in Table 19. In other Embodiments, in addition to the data provided in Table 19, the endoscopic objective optical system may also adopt any other feasible parameters as long as the requirements are met. For instance, in other embodiments, according to usage needs, D11/imgH may also be less than or equal to 1, or in other embodiments, D11/imgH may also be greater than or equal to 1.34. In other examples, (H1/f1)/(H4/f4) may also be greater than 2. In other embodiments, provided that the requirements are satisfied, other parameters of the endoscopic objective optical system such as f, FOV, imgH, and TTL may also be adjusted according to need.

**Table 19 Data of the Optical System of the Endoscope Objective Lens**

| | Embodim ent 1 | Embodim ent 2 | Embodim ent 3 | Embod iment 4 | Embodi ment 5 | Embodi ment 6 | Embodi ment 7 | Embo diment 8 | Embodi ment 9 |
|---|---|---|---|---|---|---|---|---|---|
| f | 0.750 | 0.758 | 0.826 | 0.754 | 0.753 | 0.719 | 0.709 | 0.7930 | 0.747 |
| TTL | 2.898 | 2.782 | 2.693 | 2.697 | 2.782 | 2.918 | 2.822 | 2.8150 | 2.813 |
| FOV | 140 | 140 | 140 | 140 | 140 | 140 | 150 | 125 | 140 |
| h11/h42 | 0.96 | 0.97 | 1.00 | 0.83 | 1.01 | 0.94 | 1.02 | 0.88 | 0.99 |
| D11/D4 2 | 1.04 | 1.04 | 1.08 | 0.91 | 1.06 | 1.05 | 1.11 | 0.96 | 1.04 |
| D11/im gH | 1.25 | 1.23 | 1.18 | 1.12 | 1.26 | 1.33 | 1.34 | 1.16 | 1.29 |
| (H1/f 1) / (H4/f 4) | 1.03 | 1.09 | 0.73 | 0.66 | 2.00 | 1.21 | 1.13 | 0.93 | 1.18 |
| F23/f | 0.79 | 0.79 | 0.54 | 0.72 | 0.80 | 1.20 | 0.83 | 0.77 | 0.83 |
| D11/T1 | 4.35 | 4.18 | 4.35 | 4.15 | 3.94 | 4.88 | 4.42 | 3.94 | 4.43 |

Some embodiments of endoscopes are provided, wherein the endoscopes include the endoscope objective lenses as described in any of the above embodiments.

The specific embodiments described herein are presented only to illustrate the principle of the present disclosure and shall not be construed as limiting the scope of the present disclosure. Those skilled in the art may effect variations, modifications, or equivalent substitutions based on the inventive principle disclosed herein, without departing from the spirit and scope of the disclosure.

## Claims

1. An endoscope objective lens, comprising: an objective optical system, the objective optical system comprising:
a first lens having a negative optical power, wherein a portion of an image side surface of the first lens close to an optical axis and away from an edge of the first lens is concave;
a second lens, having a positive optical power;
an aperture stop;
a third lens, having a positive optical power;
a fourth lens, having a negative optical power, wherein a portion of an object side surface of the fourth lens close to the optical axis and away from an edge of the fourth lens is concave;
wherein the first lens, the second lens, the aperture stop, the third lens and the fourth lens are arranged in sequence from an image side to an object side along the optical axis;
a chief ray height h11 on an object side surface of the first lens and a chief ray height h42 on an image side surface of the fourth lens satisfy: 0.65 ≤ h11/h42 ≤ 1.35, and/or an effective optical diameter D11 of the object side surface of the first lens and an effective optical diameter D42 of the image side surface of the fourth lens satisfy: 0.75 ≤ D11/D42 ≤ 1.25.

2. The endoscope objective lens according to claim 1, wherein
the chief ray height h11 on the object side surface of the first lens and the chief ray height h42 on the image side surface of the fourth lens satisfy: 0.8≤h11/h42≤1.2.

3. The endoscope objective lens according to claim 1, wherein
the effective optical diameter D11 of the object side surface of the first lens and the effective optical diameter D42 of the image side surface of the fourth lens satisfy: 0.85 ≤ D11/D42 ≤ 1.15.

4. The endoscope objective lens according to any one of claims 1 to 3, wherein
the effective optical diameter D11 of the object side surface of the first lens and an imaging height imgH of the objective optical system satisfy: 0.8 ≤ D11/imgH ≤ 1.42.

5. The endoscope objective lens according to claim 4, wherein
the effective optical diameter D11 of the object side surface of the first lens and the imaging height imgH of the objective optical system satisfy: 1.00 ≤ D11/imgH ≤ 1.40.

6. The endoscope objective lens according to any one of claims 1 to 3, wherein
chief ray heights at a marginal field of view on two surfaces of the first lens are h11 and h12 respectively, an average of h11 and h12 is H1, a focal length of the first lens is f1,
chief ray heights at a marginal field of view on two surfaces of the fourth lens are h41 and h42 respectively, an average of h41 and h42 is H4, a focal length of the fourth lens is f4,
whereby the objective optical system satisfies: 1 ≤ (H1/f1)/(H4/f4) ≤ 2.

7. The endoscope objective lens according to any one of claims 1 to 3, wherein
a combined focal length F23 of the second lens and the third lens, when optically treated as a lens group,
and a focal length f of the objective optical system
satisfy: 0.7 ≤ F23/f ≤ 1.2.

8. The endoscope objective lens according to any one of claims 1 to 3, wherein
an object side surface of the second lens is convex; and
an image side surface of the third lens is convex.

9. The endoscope objective lens according to any one of claims 1 to 3, wherein
a number of lenses with optical power in the objective optical system is four.

10. An endoscope, comprising the endoscope objective lens according to any one of claims 1 to 9.
